# EUROPÄISCHE PATENTSCHRIFT

(11) **EP 2 976 794 B1**
(45) Veröffentlichungstag und Bekanntmachung des Hinweises auf die Patenterteilung: **12.08.2020**
(21) Anmeldenummer: 14711169.4
(22) Anmeldetag: 21.02.2014
(51) Int. Cl.: H01L 51/46, C07D 403/14, C07D 417/14, H01L 51/42

(54) **PHOTOAKTIVES, ORGANISCHES MATERIAL FÜR OPTOELEKTRONISCHE BAUELEMENTE**
PHOTOACTIVE ORGANIC MATERIAL FOR OPTOELECTRONIC DEVICES
MATÉRIAU ORGANIQUE PHOTOACTIF POUR COMPOSANTS OPTOÉLECTRONIQUES

(30) Priorität: 21.02.2013 DE 102013101712
(43) Veröffentlichungstag der Anmeldung: 27.01.2016
(73) Patentinhaber: Heliatek GmbH, 01139 Dresden (DE)
(72) Erfinder: WEISS, Andre, 01139 Dresden (DE); HILDEBRANDT, Dirk, 89077 Ulm (DE); GERDES, Olga, 89077 Ulm (DE); MATTERSTEIG, Gunter, 89077 Ulm (DE); VETTER, Serge, 89077 Ulm (DE)
(86) Internationale Anmeldenummer: PCT/EP2014/053483
(87) Internationale Veröffentlichungsnummer: WO 2014/128281

(56) Entgegenhaltungen:
- WO-A1-2006/092134
- WO-A1-2010/139782
- WO-A1-2013/023109
- M. MANUELA M. RAPOSO ET AL: "Synthesis and Characterization of Dicyanovinyl-Substituted Thienylpyrroles as New Nonlinear Optical Chromophores", ORGANIC LETTERS, Bd. 8, Nr. 17, 1. August 2006 (2006-08-01) , Seiten 3681-3684, XP55123711, ISSN: 1523-7060, DOI: 10.1021/ol061277s
- ANA LÓPEZ-PÉREZ ET AL: "Oligopyrrole Synthesis by 1,3-Dipolar Cycloaddition of Azomethine Ylides with Bissulfonyl Ethylenes", ANGEWANDTE CHEMIE INTERNATIONAL EDITION, vol. 46, no. 48, 10 December 2007 (2007-12-10), pages 9261-9264, XP55376706, ISSN: 1433-7851, DOI: 10.1002/anie.200703258

## Beschreibung

Die Erfindung betrifft ein organisches und photoaktives Material zur Verwendung in optoelektronischen Bauelementen.

Optoelektronische Bauelemente basieren auf den optischen und elektronischen Eigenschaften von Materialien und finden heute breite Anwendung im alltäglichen Leben, wie etwa Solarzellen, LED's, TFT's. Im Allgemeinen umfassen sie alle Produkte und Verfahren, die die Umwandlung von elektronisch erzeugten Daten und Energien in Lichtemission ermöglichen oder Lichtemissionen in Energien umwandeln. Optoelektronische Bauelemente, die Lichtemission in Energien umwandeln umfassen Photodioden, die als Photovoltaikanlagen betrieben werden oder als lichtsensitive Sensoren oder Belichtungsmessern in verschiedenen Produkten wie Digitalkameras, CD-Abspielgeräte, Lichtschranken eingesetzt werden.

Optoelektronische Bauelemente aus größtenteils organischen Materialien sind für die Anwendung als LEDs (OLED) und Photovoltaikanlagen (OPV) bekannt. Die verwendeten organischen Materialien erfüllen in diesen optoelektronischen Bauelementen unterschiedliche Aufgaben wie z.B. den Ladungstransport, die Lichtemssion oder Lichtabsorption. Organische Materialien in optoelektronischen Bauelementen können dabei Polymere oder kleine Moleküle sein und in Lösung oder Emulsion durch nasschemische Prozesse wie Coaten oder Drucken oder im Vakuum durch z.B. Sublimation zu dünnen Schichten verarbeitet werden.

Eine Solarzelle wandelt Lichtenergie in elektrische Energie um. In diesem Sinne wird der Begriff "photoaktiv" als Umwandlung von Lichtenergie in elektrische Energie verstanden. Im Gegensatz zu anorganischen Solarzellen werden bei organischen Solarzellen durch das Licht nicht direkt freie Ladungsträger erzeugt, sondern es bilden sich zunächst Exzitonen, also elektrisch neutrale Anregungszustände (gebundene Elektron-Loch-Paare). Daher spielt die rekombinationsarme Diffusion von Exzitonen an die aktive Grenzfläche eine kritische Rolle bei organischen Solarzellen. Um einen Beitrag zum Photostrom zu leisten, muss daher in einer guten organischen Solarzelle die Exzitonendiffusionslänge die typische Eindringtiefe des Lichts deutlich übersteigen, damit der überwiegende Teil des Lichts genutzt werden kann.

Falls es sich bei der Absorberschicht um eine Mischschicht handelt, so übernimmt die Aufgabe der Lichtabsorption entweder nur eine der Komponenten oder auch beide. Der Vorteil von Mischschichten ist, dass die erzeugten Exzitonen nur einen sehr kurzen Weg zurücklegen müssen bis sie an eine Domänengrenze gelangen, wo sie getrennt werden. Der Abtransport der Elektronen bzw. Löcher erfolgt getrennt in den jeweiligen Materialien. Da in der Mischschicht die Materialien überall miteinander im Kontakt sind, ist bei diesem Konzept entscheidend, dass die getrennten Ladungen eine lange Lebensdauer auf dem jeweiligen Material besitzen und von jedem Ort aus geschlossene Perkolationspfade für beide Ladungsträgersorten zum jeweiligen Kontakt hin vorhanden sind.

Ein wichtiger Faktor in der Verbesserung der oben genannten Solarzellen liegt in der Weiterentwicklung der organischen Schichten. Für die Absorberschichten, speziell auf dem Gebiet kleiner Moleküle, sind in den letzten 5 Jahren wenige neue Materialien bekannt geworden.

In der WO2006092134A1 werden Verbindungen offenbart, die über einen Akzeptor-Donor-Akzeptor-Aufbau verfügen, wobei der Donorblock ein ausgedehntes π-System besitzt.

In der WO2013023109A1 werden Heteroaryl-Verbindungen zur Verwendung in organischen optoelektronischen Bauelementen offenbart.

In der DE60205824T2 werden Thienothiophenderivate offenbart, die mit weiteren Aromaten ein π-System bilden und an beiden Seiten von Alkylgruppen eingerahmt sind, und deren Verwendung in organischen Halbleitern.

Raposo et al. (Organic Letters, 2006, Vol.8, No.17, 3681-3684) offenbart die Synthese und Charakterisierung von Dicyanovinylsubstituierter Thienopyrrole als nicht-lineare optische Chromophore.

Lopez-Perez et al. (Angew. Chem. Int. Ed., 2007, 46, 9261-9264) offenbart die Synthese von Oligopyrrolen durch eine 1,3-dipolare Cycloaddition von Azomethine-Yliden mit Bissulfonyl-Ethylenen.

In der WO2009051390 werden Thiophen basierte Akzeptor-Donator Farbstoffe für den Einsatz in farbstoffsensitiven Solarzellen offenbart.

In der WO002008145172A1 werden neuartige Phthalocyanine zur Verwendung in Solarzellen vorgestellt.

In der WO2010139782A1 wird ein lichtabsorbierendes organisches Bauelement offenbart, wobei ein photoaktiver Bereich ein aus Monomeren bestehendes pi-konjugiertes Oligomer aufweist.

In der US7655809B2 werden Verbindungen aus kondensierten Kohlenstoffzyklen in Reihe und deren Verwendung als organische Halbleiter offenbart.

In der WO 2006111511A1 und WO2007116001A2 werden Rylentetracarbonsäurederivate zur Verwendung als aktive Schicht in Photovoltaik offenbart.

Dagegen sind verschiedene Polymere zur Verwendung als aktive Schichten in organischer Photovoltaik bekannt, beispielweise offenbart in WO 2008088595A2, EP2072557A1 oder US20070112171A1. Diese sind im Allgemeinen nicht verdampfbar, sondern werden in flüssiger Form zu dünnen Schichten verarbeitet.

Um weitere Effizenzsteigerungen bei organischen Solarzellen zu erzielen, ist es nötig, in einem größeren Spektrum des Sonnenlichtes zu absorbieren. Dafür ist eine Kombination verschiedener Absorbermaterialien notwendig.

Aufgabe dieser Erfindung ist es also Materialien bereitzustellen, welche als aktive Schichten in optoelektronischen Bauelementen eingesetzt werden können und die Nachteile des Standes der Technik überwinden.

Die Aufgabe wird durch die unabhängigen Ansprüche der vorliegenden Anmeldung gelöst. Bevorzugte Ausführungsformen ergeben sich aus den Unteransprüchen.

Erfindungsgemäß wird eine Verbindung der allgemeinen Formel (I) 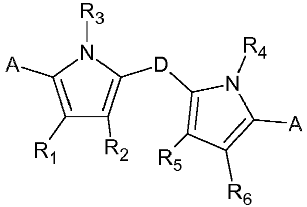
mit R₃ und R₄ unabhängig ausgesucht aus Alkyl, Aryl oder substituiertem Alkyl mit 1-26 C-Atomen, wobei maximal 2 C-Atome ersetzt sind durch N, O oder S oder maximal 5 H-Atome ersetzt sind durch F, Cl, Br oder CN,
R1, R2, R5 und R6 unabhängig voneinander ausgesucht aus H, Alkyl oder O-Alkyl, wobei R1 und R2 und/oder R5 und R6 einen Ring bilden können, und
wobei A ausgesucht ist aus: 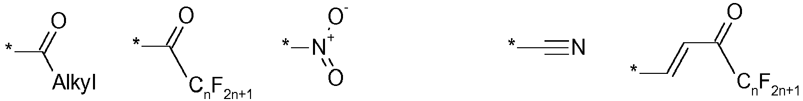 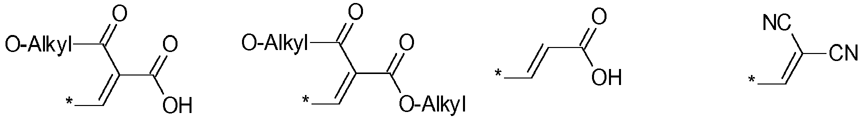 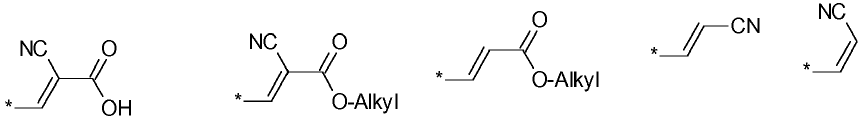 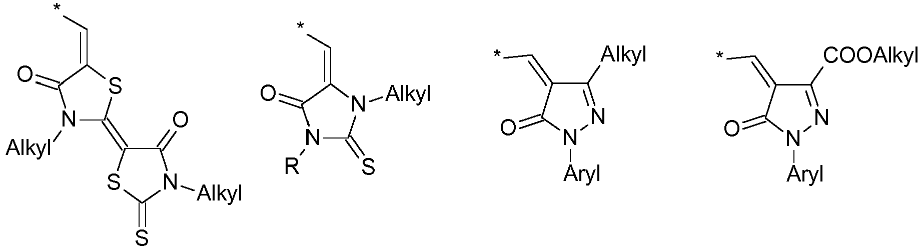 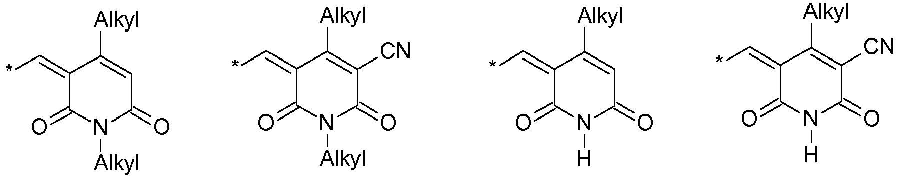 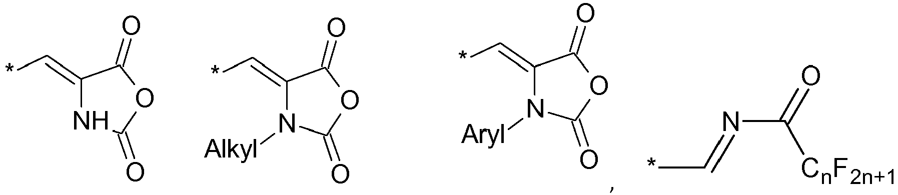 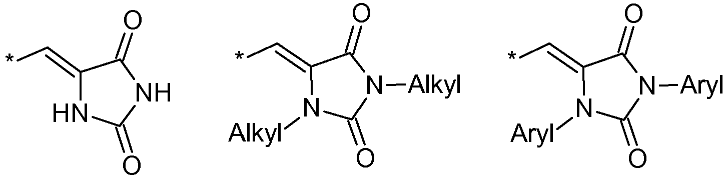
wobei n eine ganze Zahl von 1 bis 5 ist,
wobei D eine Einheit aus linear konjugierten aromatischen 5- und/oder 6-Ringen ist, die anneliert sein können, und
wobei D ausgesucht ist aus: 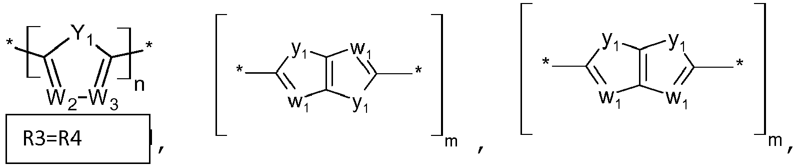 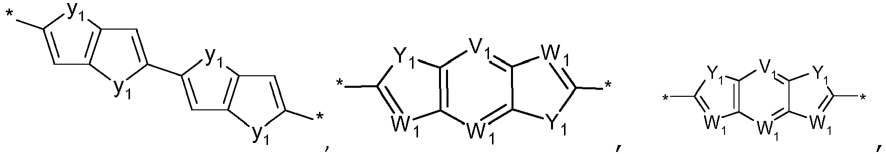 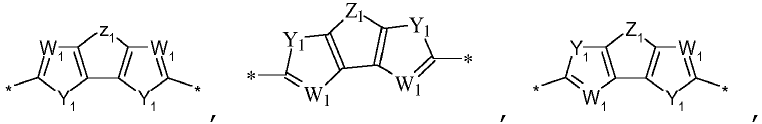 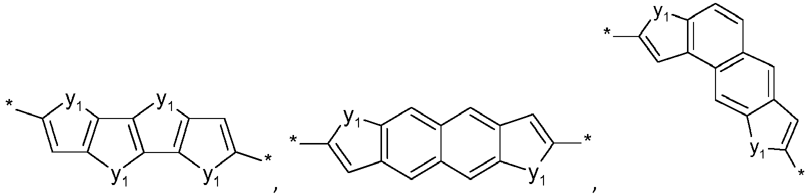
wobei Y₁ und Z₁ unabhängig ausgewählt sind aus: O, S, Se, Si(RR'), C(RR') und N(R), mit R und R' unabhängig voneinander ausgewählt aus H, Alkyl oder Aryl und
W₁ und V₁ unabhängig ausgewählt ist aus: N und C-R`, mit R` unabhängig voneinander ausgewählt aus H, Alkyl, O-Alkyl, S-Alkyl oder Aryl
W₂ jeweils unabhängig ausgewählt ist aus: N und C-R9 mit R9 = H, Halogen, Alkyl, Alkenyl, Alkinyl, jeweils linear oder verzweigt, OR', SR', SiR'3, NR'2 mit R` ausgesucht aus Alkyl oder Aryl,
W₃ jeweils unabhängig ausgewählt ist aus: N und C-R10 mit R10 = H, Halogen, Alkyl, Alkenyl, Alkinyl, jeweils linear oder verzweigt, OR', SR`, SiR'3, NR'2 mit R' ausgesucht aus Alkyl oder Aryl,
wobei R9 und R10 einen Ring bilden können, und
n = 1, 2 oder 3 und m = 1 oder 2 ist.

In einer Ausführungsform der Erfindung ist A ausgesucht aus: 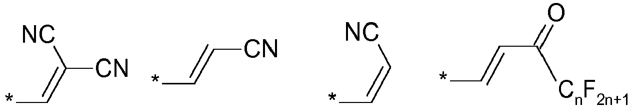 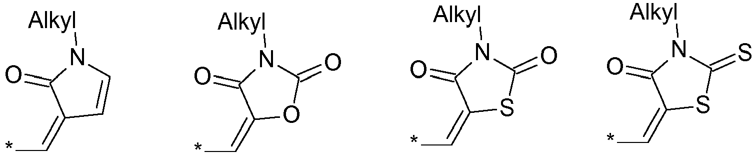 wobei n eine ganze Zahl von 1 bis 5 sein kann.

Dabei kann A beidseitig gleich oder unterschiedlich sein, bevorzugt besitzt A beidseitig eine ähnliche Akzeptorstärke. Besonders bevorzugt ist A beidseitig gleich.

In einem Ausführungsbeispiel der Erfindung sind R3 und R4 der allgemeinen Formel I H und die Akzeptorgruppen A sind so ausgesucht, dass sie in einer Wasserstoffbrückenbindung zum Pyrrol-NH einen 5-Ring, 6-Ring oder 7-Ring bilden. Mögliche Akzeptorgruppen sind folgende:

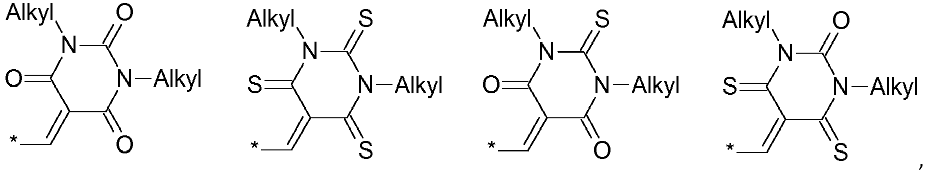

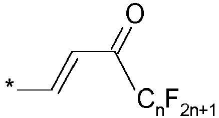

In einem Ausführungsbeispiel der Erfindung sind R3 und R4 der allgemeinen Formel I unabhängig voneinander Alkyl, Aryl oder substituiertem Alkyl mit 1-26 C-Atomen, wobei maximal 2 C-Atome ersetzt sind durch N, O oder S oder maximal 5 H-Atome ersetzt sind durch F, Cl, Br oder CN,

R1, R2, R5 und R6 unabhängig voneinander ausgesucht aus H, Alkyl oder O-Alkyl, wobei R1 und R2 und/oder R5 und R6 einen Ring bilden können, und die Akzeptorgruppen A sind so ausgesucht, dass eine sterische Hinderung zum Substituenten des Pyrrol-Stickstoffes minimiert wird. Mögliche Akzeptorgruppen sind folgende:

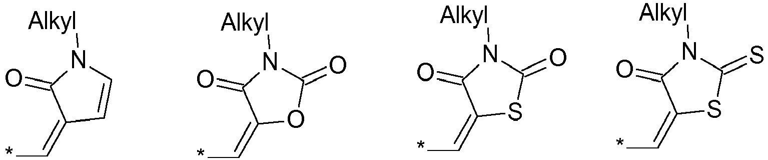

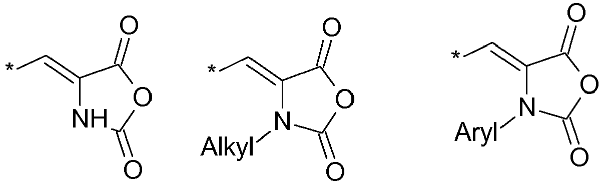

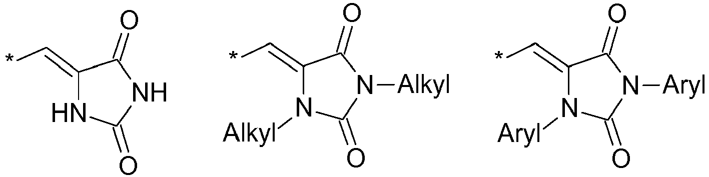

In einer Ausführungsform der Erfindung sind die Akzeptorgruppen A jeweils gleich und ausgesucht aus cis-CH=CHCN, trans-CH=CHCN oder CH=C(CN)₂.

In einer weiteren Ausführungsform der Erfindung ist D ausgesucht aus: 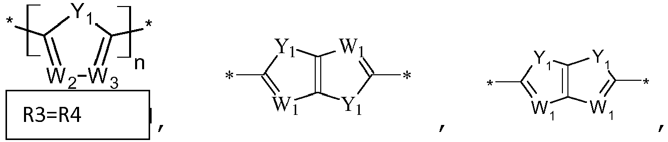 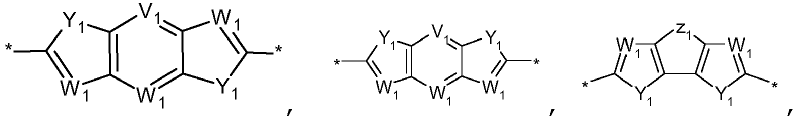 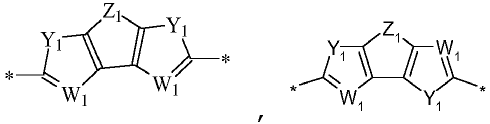
wobei Y₁ und Z₁ unabhängig ausgewählt sind aus: O, S, Se, Si(RR'), C(RR') und N(R), mit R und R' unabhängig voneinander ausgewählt aus H, Alkyl oder Aryl und
W₁ und V₁ unabhängig ausgewählt ist aus: N und C-R`, mit R` unabhängig voneinander ausgewählt aus H, Alkyl, O-Alkyl, S-Alkyl oder Aryl
W₂ jeweils unabhängig ausgewählt ist aus: N und C-R9 mit R9 = H, Halogen, Alkyl, Alkenyl, Alkinyl, jeweils linear oder verzweigt, OR', SR', SiR'3, NR'2 mit R` ausgesucht aus Alkyl oder Aryl,
W₃ jeweils unabhängig ausgewählt ist aus: N und C-R10 mit R10 = H, Halogen, Alkyl, Alkenyl, Alkinyl, jeweils linear oder verzweigt, OR', SR', SiR'3, NR'2 mit R' ausgesucht aus Alkyl oder Aryl,
wobei R9 und R10 einen Ring bilden können, und n = 1, 2 oder 3 ist.

Für eine Verarbeitung der erfindungsgemäßen Materialien mit lösungsmittelbasierten Methoden, wie Drucken, Coaten, Aufschleudern oder ähnliches ist es sinnvoll die Akzeptorgruppe A und etwaige Seitenketten R so zu wählen, dass eine ausreichende Löslichkeit gegeben ist.

Für eine Verarbeitung der erfindungsgemäßen Materialien durch Verdampfen, z.B. im Vakuum mit oder ohne Trägergas, ist es sinnvoll, dass die Moleküle eine Molmasse von maximal 1000 g/mol besitzt.

In einer Ausführungsform der Erfindung wird eine Verbindung der allgemeinen Formel (I) 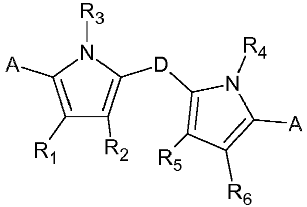
mit R₃ und R₄ unabhängig ausgesucht aus Alkyl, Aryl oder substituiertem Alkyl mit 1-26 C-Atomen, wobei maximal 2 C-Atome ersetzt sind durch N, O oder S oder maximal 5 H-Atome ersetzt sind durch F, Cl, Br oder CN,
R1, R2, R5 und R6 unabhängig voneinander ausgesucht aus H, Alkyl oder O-Alkyl, wobei R1 und R2 und/oder R5 und R6 einen Ring bilden können, und wobei A ausgesucht ist aus: 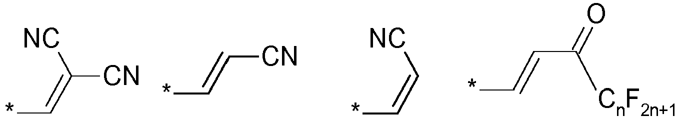 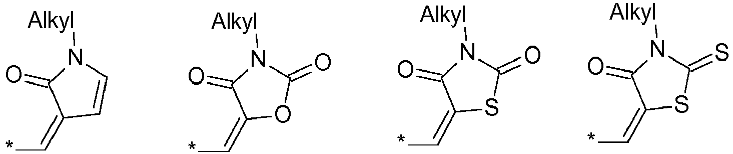
wobei n eine ganze Zahl von 1 bis 5 sein kann,
und D ausgesucht ist aus: 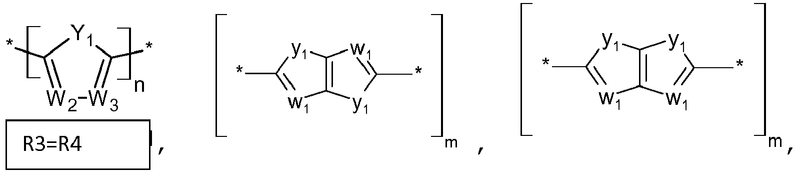 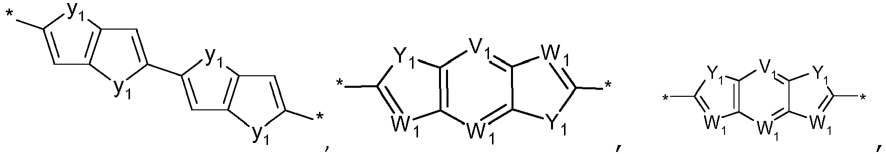 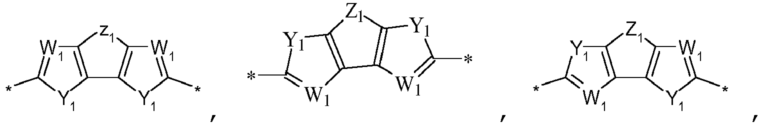 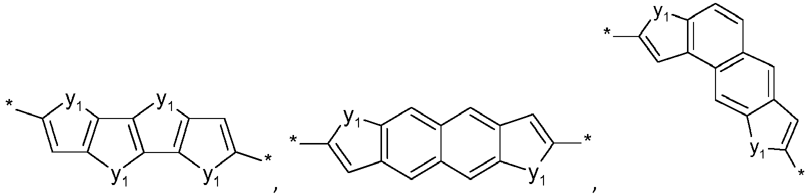
wobei Y₁ und Z₁ unabhängig ausgewählt sind aus: O, S, Se, Si(RR'), C(RR') und N(R), mit R und R' unabhängig voneinander ausgewählt aus H, Alkyl oder Aryl und
W₁ und V₁ unabhängig ausgewählt ist aus: N und C-R', mit R', unabhängig voneinander ausgewählt aus H, Alkyl, O-Alkyl, S-Alkyl oder Aryl
W₂ jeweils unabhängig ausgewählt ist aus: N und C-R9 mit R9 = H, Halogen, Alkyl, Alkenyl, Alkinyl, jeweils linear oder verzweigt, OR', SR', SiR'3, NR'2 mit R` ausgesucht aus Alkyl oder Aryl,
W₃ jeweils unabhängig ausgewählt ist aus: N und C-R10 mit R10 = H, Halogen, Alkyl, Alkenyl, Alkinyl, jeweils linear oder verzweigt, OR', SR', SiR'3, NR'2 mit R' ausgesucht aus Alkyl oder Aryl,
wobei R9 und R10 einen Ring bilden können, und
n = 1, 2 oder 3 und m = 1 oder 2 ist.

In einer Ausführungsform der Erfindung wird eine Verbindung der allgemeinen Formel (I) 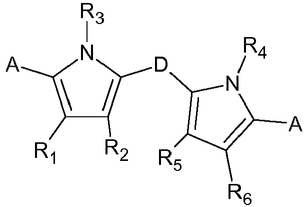
mit R₃ und R₄ unabhängig ausgesucht aus Alkyl, Aryl oder substituiertem Alkyl mit 1-26 C-Atomen, wobei maximal 2 C-Atome ersetzt sind durch N, O oder S oder maximal 5 H-Atome ersetzt sind durch F, Cl, Br oder CN,
R1, R2, R5 und R6 unabhängig voneinander ausgesucht aus H, Alkyl oder O-Alkyl, wobei R1 und R2 und/oder R5 und R6 einen Ring bilden können, und A ist jeweils gleich und ausgesucht aus cis-CH=CHCN, trans-CH=CHCN oder CH=C(CN)₂, vorgeschlagen,
wobei die Gruppe D ausgesucht ist aus: 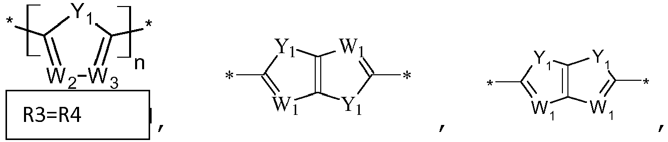 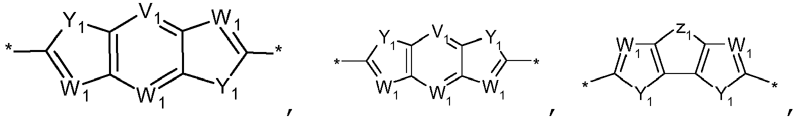 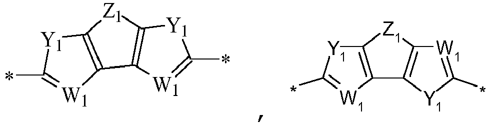
wobei Y₁ und Z₁ unabhängig ausgewählt sind aus: O, S, Se, Si(RR'), C(RR') und N(R), mit R und R' unabhängig voneinander ausgewählt aus H, Alkyl oder Aryl und
W₁ und V₁ unabhängig ausgewählt ist aus: N und C-R', mit R` unabhängig voneinander ausgewählt aus H, Alkyl, O-Alkyl, S-Alkyl oder Aryl
W₂ jeweils unabhängig ausgewählt ist aus: N und C-R9 mit R9 = H, Halogen, Alkyl, Alkenyl, Alkinyl, jeweils linear oder verzweigt, OR', SR`, SiR'3, NR'2 mit R` ausgesucht aus Alkyl oder Aryl,
W₃ jeweils unabhängig ausgewählt ist aus: N und C-R10 mit R10 = H, Halogen, Alkyl, Alkenyl, Alkinyl, jeweils linear oder verzweigt, OR', SR', SiR'3, NR'2 mit R' ausgesucht aus Alkyl oder Aryl,
wobei R9 und R10 einen Ring bilden können, und
n = 1, 2 oder 3 ist.

Für die Gruppe D ausgewählt aus 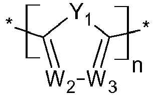 gilt, dass R3 und R4 nicht H sind.

Für die Gruppe D ausgewählt aus 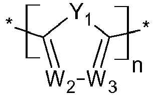 gilt, dass für n > 1, die Substituenten W₂ und W₃ jeweils gleich oder verschieden sein können, so dass folgende Kombinationen für die Gruppe D realisierbar sind:

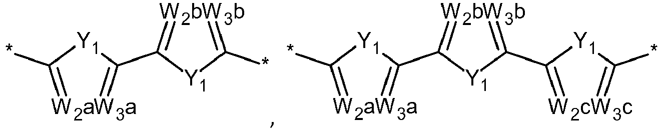

Mit W₂a, W₂b und W₂c jeweils unabhängig voneinander ausgesucht aus N und C-R9 mit R9 = H, Halogen, Alkyl, Alkenyl, Alkinyl, jeweils linear oder verzweigt, OR', SR', SiR'3, NR'2 mit R' ausgesucht aus Alkyl oder Aryl,
und W₃a, W₃b und W₃c jeweils unabhängig voneinander ausgesucht aus N und C-R10 mit R10 = H, Halogen, Alkyl, Alkenyl, Alkinyl, jeweils linear oder verzweigt, OR', SR', SiR'3, NR'2 mit R' ausgesucht aus Alkyl oder Aryl,
wobei R9 und R10 einen Ring bilden können.

Die mit dem Stern* gekennzeichneten Bindungen, deuten die Bindungen zu weiteren Gruppen in den Verbindungen an.

In einer Ausführungsform der Erfindung weisen die erfindungsgemäßen Verbindungen nach der Formel (I) zwei Elektronenakzeptorgruppen A auf, die gleich sind und cis-Monocyanovinyl, trans-Monocyanovinyl oder Dicyanovinyl sein können, und den ausgedehnten Elektronendonorblock flankieren.

In einer Ausführunsgform der Erfindung besitzen die erfindungsgemäßen Verbindungen einen Elektronendonorblock, der mindestens 6 und maximal 12, bevorzugt maximal 10 linear konjugierte Doppelbindungen besitzt.

Unter einem "Elektronendonorblock" soll in vorliegender Anmeldung der ganze Mittelblock zwischen den Akzeptoren A nach der allgemeinen Formel (I) zählen, einschließlich der beiden außenständigen Pyrrolringe. Der Elektronendonorblock besitzt ein vollständig linear durchkonjugiertes π-System.

Unter "Alkyl" soll in vorliegender Anmeldung Alkylketten verstanden werden mit einer Länge von 1 bis 26 C-Atomen (C1-C26-Alkyl), wobei diese Ketten sowohl geradlinig, n-Alkyl, als auch verzweigt, iso-Alkyl, sein können. Bevorzugt soll unter "Alkyl" in vorliegender Anmeldung eine Alkylkette mit einer Länge von 1 bis 10 C-Atomen (C1-C10-Alkyl), wobei diese Ketten sowohl geradlinig, n-Alkyl, als auch verzweigt, iso-Alkyl, sein können, verstanden werden.

Ebenso sollen unter "S-Alkyl" in vorliegender Arbeit Thioalkylether verstanden werden, wobei der Schwefel S immer an Position 1 ist und Alkyl mit einer Länge von 1 bis 26 C-Atomen (C1-C26-S-Alkyl) vorhanden ist, wobei diese Ketten sowohl geradlinig, n-S-Alkyl, als auch verzweigt, iso-S-Alkyl, sein können. Bevorzugt soll dabei die Alkylkette des "S-Alkyl" in vorliegender Anmeldung eine Länge von 1 bis 10 C-Atomen (C1-C10-S-Alkyl) haben, wobei diese Ketten sowohl geradlinig, n-S-Alkyl, als auch verzweigt, iso-S-Alkyl, sein können.

Ebenso sollen unter "O-Alkyl" in vorliegender Arbeit Alkylether verstanden werden, wobei der Sauerstoff O immer an Position 1 ist und Alkyl mit einer Länge von 1 bis 26 C-Atomen (C1-C26-O-Alkyl) vorhanden ist, wobei diese Ketten sowohl geradlinig, n-O-Alkyl, als auch verzweigt, iso-O-Alkyl, sein können. Bevorzugt soll dabei die Alkylkette des "O-Alkyl" in vorliegender Anmeldung eine Länge von 1 bis 10 C-Atomen (C1-C10-O-Alkyl) haben, wobei diese Ketten sowohl geradlinig, n-O-Alkyl, als auch verzweigt, iso-O-Alkyl, sein können.

Unter "Alkenyl" soll in vorliegender Anmeldung Alkenylketten verstanden werden mit einer Länge von 2 bis 26 C-Atomen (C2-C26-Alkenyl), wobei mindestens eine C-C-Doppelbindung in der Kette vorhanden ist und die Ketten sowohl geradlinig, n-Alkenyl, als auch verzweigt, iso-Alkenyl, sein können. Bevorzugt soll unter "Alkenyl" in vorliegender Anmeldung Alkenylketten verstanden werden mit einer Länge von 2 bis 10 C-Atomen (C2-C10-Alkenyl), wobei mindestens eine C-C-Doppelbindung in der Kette vorhanden ist und die Ketten sowohl geradlinig, n-Alkenyl, als auch verzweigt, iso-Alkenyl, sein können, verstanden werden.

Unter "Alkinyl" soll in vorliegender Anmeldung Alkinylketten verstanden werden mit einer Länge von 2 bis 26 C-Atomen (C2-C26-Alkinyl), wobei mindestens eine C-C-Dreifachbindung in der Kette vorhanden ist und die Ketten sowohl geradlinig, n-Alkinyl, als auch verzweigt, iso-Alkinyl, sein können. Bevorzugt soll unter "Alkinyl" vorliegender Anmeldung Alkinylketten verstanden werden mit einer Länge von 2 bis 10 C-Atomen (C2-C10-Alkinyl), wobei mindestens eine C-C-Dreifachbindung in der Kette vorhanden ist und die Ketten sowohl geradlinig, n-Alkinyl, als auch verzweigt, iso-Alkinyl, sein können, verstanden werden.

Unter "Aryl" werden Arylreste verstanden mit 5-20 aromatischen Ringatomen, die mit einem oder mehreren Resten R substituiert sein können, wobei R gleich oder verschieden ausgewählt ist aus der Gruppe bestehend aus H, D, F, Cl, Br, I, CN, NO2, einer geradkettigen Alkyl-, Alkoxy-, Thioalkylgruppe mit 1-26 C-Atomen oder einer verzweigten oder cyclischen Alkyl-, Alkoxy-, Thioalkylgruppe mit 3-10 C-Atomen oder einer Alkenyl- oder einer Alkinylgruppe mit 2-26 C-Atomen, wobei ein oder mehrere H-Atome durch D, F, Cl, Br, I oder CN ersetzt sein können. Die Summe aller Substituenten ist dabei bevorzugt nicht mehr als 26 Atome außer H.

In einer Ausführungsform der Erfindung sind R1, R2, R5 und R6 H.

In einer Ausführungsform der Erfindung ist in der Verbindung der allgemeinen Formel (I) D ausgesucht aus 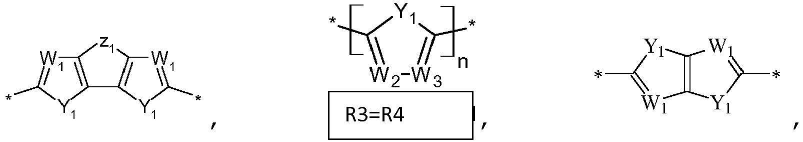 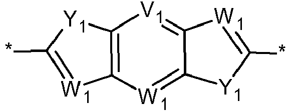
wobei Y₁ unabhängig ausgewählt ist aus: O, S, Se und N(R), mit R ausgewählt aus Alkyl oder Aryl,
Z₁ unabhängig ausgewählt ist aus: S, Se, Si(RR'), C(RR') und N(R), mit R und R' unabhängig voneinander ausgewählt aus Alkyl oder Aryl,
W₁ und V₁ unabhängig ausgewählt ist aus: C-R, mit R unabhängig ausgewählt aus H, Alkyl oder Aryl,
W₂ ausgewählt ist aus: N und C-R9 mit R9 = H, Halogen, Alkyl, Alkenyl, Alkinyl, jeweils linear oder verzweigt, OR', SR', SiR'3, NR'2 mit R' ausgesucht aus Alkyl oder Aryl,
W₃ ausgewählt ist aus: N und C-R10 mit R10 = H, Halogen, Alkyl, Alkenyl, Alkinyl, jeweils linear oder verzweigt, OR', SR', SiR'3, NR'2 mit R' ausgesucht aus Alkyl oder Aryl,
wobei R9 und R10 einen Ring bilden können, und
n = 1, 2 oder 3 ist.

In einer Ausführungsform der Erfindung ist in der Verbindung der allgemeinen Formel (I) R3 und R4 ausgesucht aus H, eine Alkylkette mit 1-10 C-Atomen, geradlinig oder verzweigt, wobei ein C-Atom durch ein O, S oder CN ersetzt sein kann oder Phenyl und D ausgesucht aus 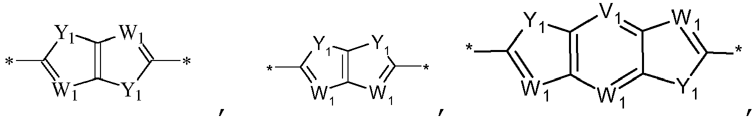 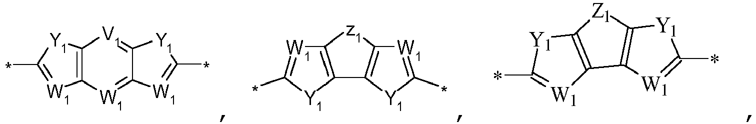 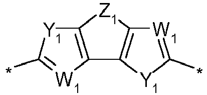
wobei Y₁ und Z₁ unabhängig ausgewählt ist aus: O, S, Se, Si(RR'), C(RR') und N(R), mit R und R' unabhängig voneinander ausgewählt aus H, Alkyl oder Aryl und
W₁ und V₁ unabhängig ausgewählt ist aus: N und C-R', mit R' unabhängig voneinander ausgewählt aus H, Alkyl, O-Alkyl, S-Alkyl oder Aryl
oder
R3 und R4 ausgesucht aus einer Alkylkette mit 1-10 C-Atomen, geradlinig oder verzweigt, wobei ein C-Atom durch ein O, S oder CN ersetzt sein kann oder Phenyl und D ausgesucht aus 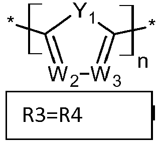
mit W₂ jeweils unabhängig ausgewählt ist aus: N und C-R9 mit R9 = H, Halogen, Alkyl, Alkenyl, Alkinyl, jeweils linear oder verzweigt, OR', SR`, SiR'3, NR'2 mit R' ausgesucht aus Alkyl oder Aryl,
W₃ jeweils unabhängig ausgewählt ist aus: N und C-R10 mit R10 = = H, Halogen, Alkyl, Alkenyl, Alkinyl, jeweils linear oder verzweigt, OR', SR`, SiR'3, NR'2 mit R' ausgesucht aus Alkyl oder Aryl,
wobei R9 und R10 einen Ring bilden können, und
n = 1, 2 oder 3 ist.

Bevorzugte erfindungsgemäße Verbindungen der allgemeinen Formel (I) sind nachfolgend dargestellt:

| No | Struktur | Lambda max (Lsg) | Variante (I, II, oder III) |
|---|---|---|---|
| 1 | 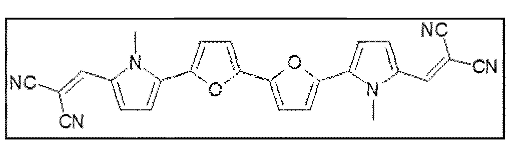 | | |
| 2 | 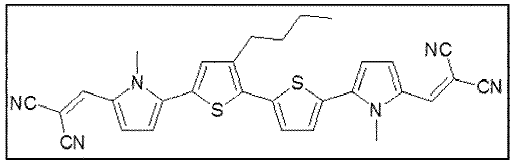 | 477 nm (DCM) | II |
| 3 | 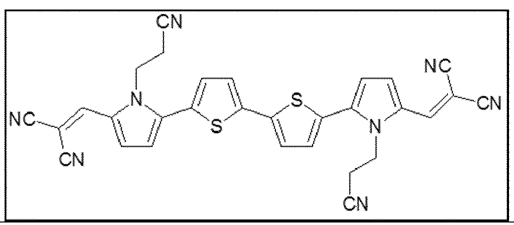 | | |
| 4 | 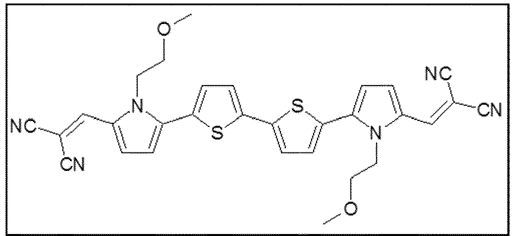 | | |
| 5 | 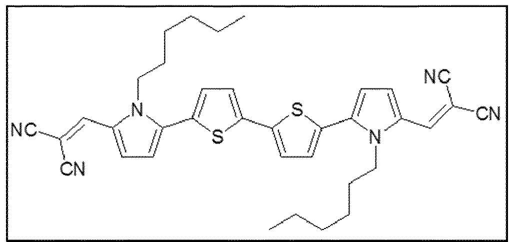 | | |
| 6 | 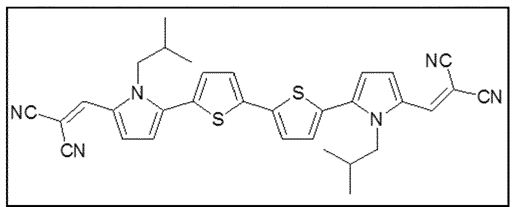 | 474 nm (DCM) | I |
| 7 | 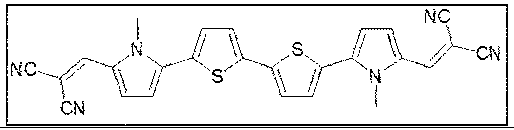 | 481 nm (DCM) | II |
| 8 | 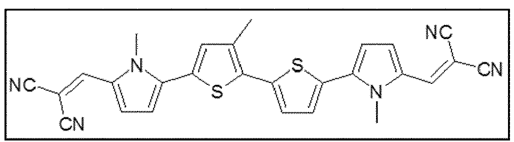 | 485 nm (DMF) | II |
| 9 | 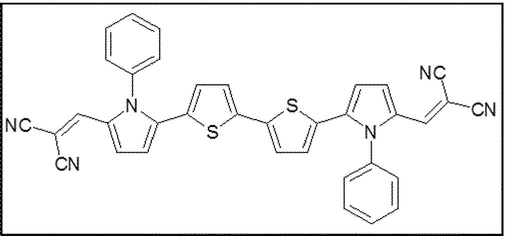 | 510 nm (DCM) | I |
| 10 | 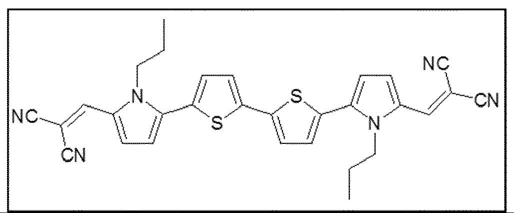 | 477 nm (DCM) | I |
| 11 | 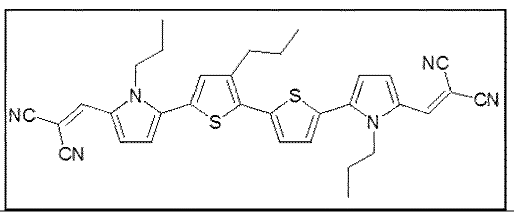 | 468 nm (DCM) | I |
| 12 | 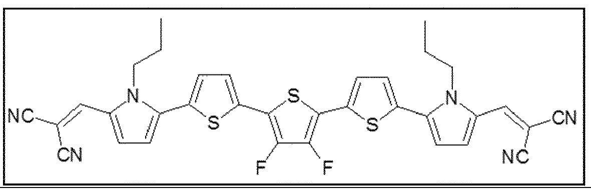 | 481 nm (DCM) | I |
| 13 | 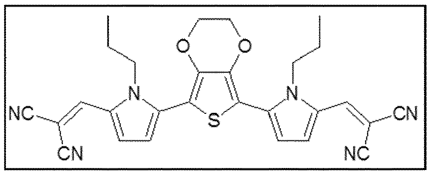 | 466 nm (DCM) | I |
| 14 | 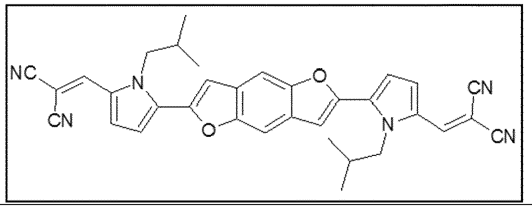 | 489 nm (DCM) | II |
| 15 | 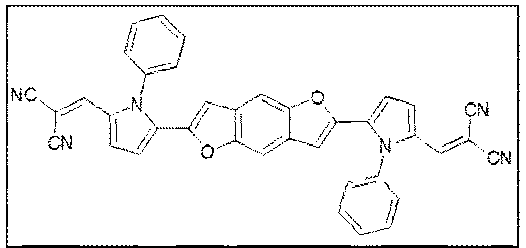 | 476 nm (DCM) | II |
| 16 | 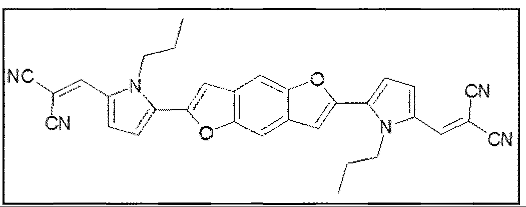 | 496 nm (DCM) | I |
| 17 | 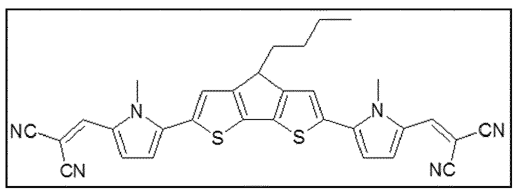 | | II |
| 18 | 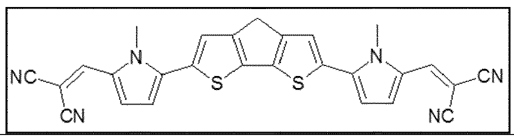 | 454 nm (DCM) | II |
| 19 | 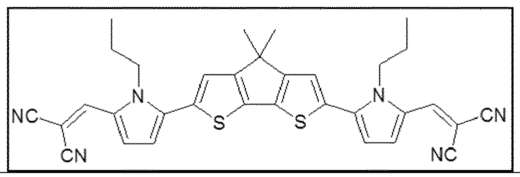 | 520 nm (DCM) | I |
| 20 | 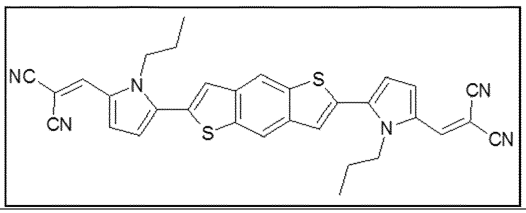 | 447 nm (DCM) | I |
| 21 | 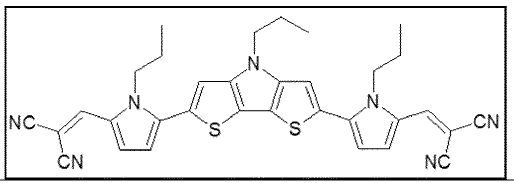 | 522 nm (DCM) | I |
| 22 | 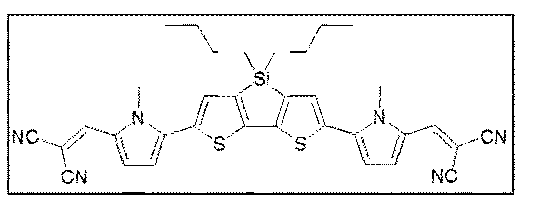 | 512 nm (DCM) | II |
| 23 | 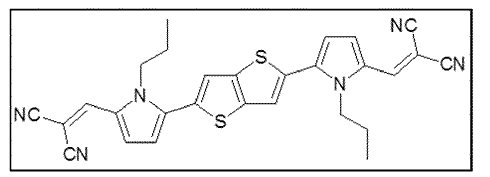 | 468 nm (DCM) | III |
| 24 | 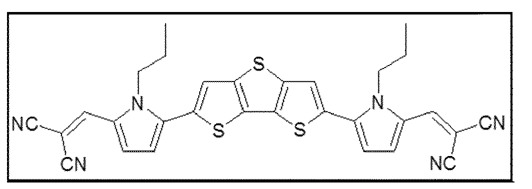 | 477 nm (DCM) | III |

In einer Ausführungsform der Erfindung werden die erfindungsgemäßen Verbindungen der allgemeinen Formel (I) als Lichtabsorber in einer Solarzelle verwendet. Besonders bevorzugt werden die erfindungsgemäßen Verbindungen der allgemeinen Formel (I) in einer Tandem- oder Mehrfachsolarzelle verwendet, wobei mindestens ein weiteres Absorbermaterial, welches in einem anderen spektralen Bereich des Lichtes absorbiert, vorhanden ist.

In einer Ausführungsform der Erfindung werden die Verbindungen der allgemeinen Formel (I) als Donatoren in einem photoaktiven Heteroübergang eingesetzt mit einem Akzeptor wie z.B. aus der Gruppe der Fullerene.

In einer Ausführungsform der Erfindung sind in einem photoaktiven Heteroübergang entweder 2 Donatoren und ein Akzeptor oder 2 Akzeptoren und ein Donator vorhanden, wobei mindestens eine Donator und/oder Akzeptor eine erfindungsgemäße Verbindung enthält.

Unter "photoaktiven Heteroübergang" wird in vorliegender Erfindung die Grenzschicht zwischen zwei unterschiedlichen Materialien verstanden, wobei das eine Material als Elektronenakzeptor fungiert und ein anderes Material als Elektronendonator fungiert. Dies beinhaltet die Grenzschicht zwischen zwei angrenzenden Schichten (flacher Heteroübergang) und die Grenzschichten in einer Mischschicht (Volumen-Heteroübergang) oder eine Kombination aus beiden.

Erstaunlicherweise wurde gefunden, dass durch das Einfügen von Pyrrolen in Moleküle der Bauweise Akzeptor-Donor-Akzeptor an den beiden äußeren Positionen des Donorblocks eine Blauverschiebung der Absorption in Lösung als auch im aufgedampften Film vorhanden ist verglichen zu baugleichen Molekülen mit Thiophenen, Selenophenen, Furanen oder Phenylen in dieser Position. Dieser unvorhersehbare Effekt führt zu einer Verbindungsklasse, deren Absorptionsmaximum im spektralen Bereich von 400 bis 600 nm liegt und über ausreichende Leitfähigkeit für die Verwendung in optoelektronischen Bauelementen verfügt.

Die Absorption wird in Lösung nach folgender Methode ermittelt:
Von der zu untersuchenden Substanz werden vier unterschiedlich konzentrierte molare Lösungen in einem geeignetem Lösemittel, typischerweise in Dichlormethan (DCM), hergestellt und in einem UV-VIS-Spektrometer gemessen. Die Konzentration der Lösungen befindet sich normaler weise in einem Bereich von 10⁻⁷-10⁻⁵ mol/L. Aus den vier unabhängig gemessenen molaren Extinktionskoeffizienten wird der durchschnittliche molare Extinktionskoeffizient bestimmt.

Die Absorption wird im Film nachfolgender Methode ermittelt: Von der zu untersuchenden Substanz wird ein Film von 30nm auf Quarz in einer Vakuumkammer mittels Verdampfung/Sublimation des Materials abgeschieden und die Absorption des Films in einem UV-VIS-Spektrometer gemessen.

In vorliegender Anmeldung sollen die Absorptionsdaten der Materialien in Lösung relevant sein.

Absobermaterialien für Solarzellen müssen Licht in der richtigen Wellenlänge absorbieren, energetisch in einem photoaktiven Heteroübergang an einen Donator angepasst sein und über ausreichende Leitfähigkeit verfügen.

Ein Problem der Position von Pyrrol an den äußeren Seiten des Donorblocks und in direkter Nachbarschaft zum Akzeptor verglichen mit z.B. Thiophen wie im Stand der Technik üblich, liegt in der Dreibindigkeit vom Stickstoff. So kann es zwischen dem Substituenten am Stickstoff des Pyrrols und der Akzeptorgruppe zu sterischen Hinderungen kommen, die dazu führen, dass das komplette Molekül nicht mehr planar ist.

Durch die Wahl der Akzeptorgruppen in Abhängigkeit zu dem Substituenten am Pyrrol-Stickstoff werden planare Moleküle erhalten.

Mit Wasserstoff an den äußeren Pyrrolen sind Akzeptorgruppen sinnvoll, die Wasserstoffbrückenbindungen eingehen können, wie z.B.

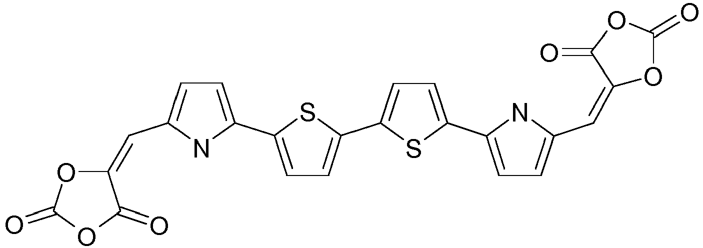

Kalkulationen mit PM3 zeigen eine planare Struktur:

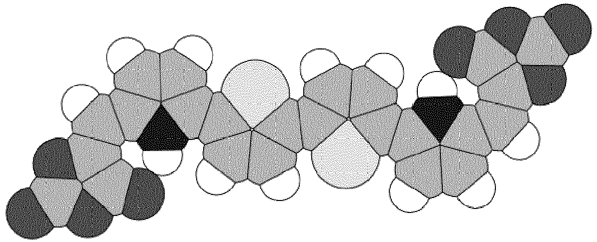

In ähnlicher Weise sollten folgende Akzeptorgruppen funktionieren: 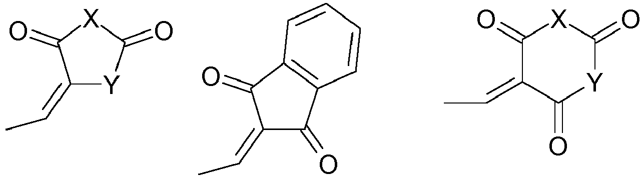 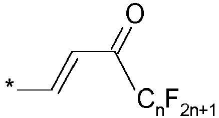 wobei X und Y unabhängig voneinander ausgesucht sind aus O, S, N-H und N-Alkyl und n eine ganze Zahl von 1 bis 5.

Eine weitere Kalkulation mit PM3 zeigt folgende Verbindung als planares Molekül:

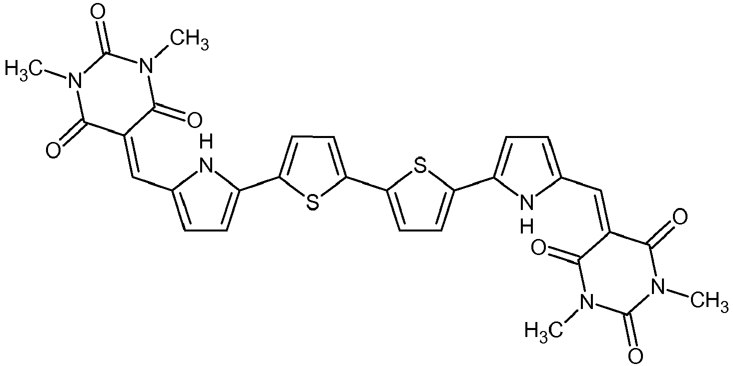

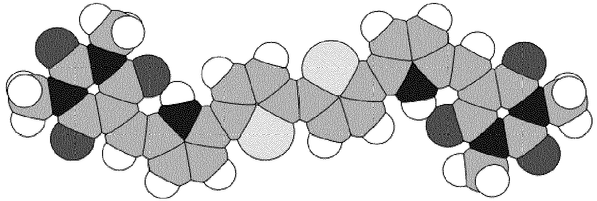

Ist eine Alkylgruppe am Stickstoff der äußeren Pyrrole ist die Verwendung von Akzeptorgruppen mit einer sterisch nicht anspruchsvollen Position benachbart zur verbindenden Doppelbindung der Akzeptorgruppe, wie z.B.:

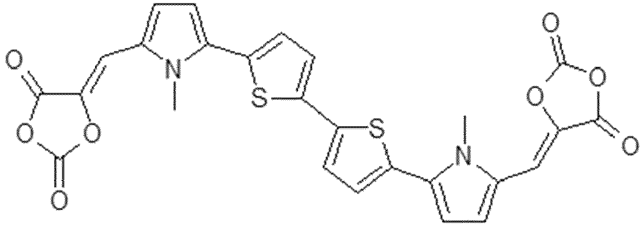

Kalkulationen mit PM3 zeigen ein planares Molekül, wobei die Alkylgruppe des Pyrrolstickstoffes und die verbindende Doppelbindung der Akzeptorgruppe in die gleiche Richtung zeigen.

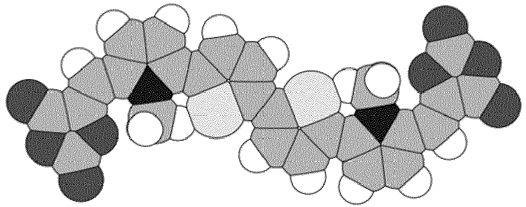

Dies gilt für alle Akzeptorgruppen der folgenden Formel: 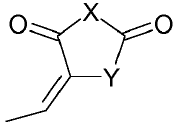 wobei X ausgewählt ist aus O, S, N-H und N-Alkyl und Y ausgesucht ist aus O und S.

Eine planare Ausrichtung mit entgegengesetzter Richtung der Alkylgruppe des Pyrrol-Stickstoffs und der verbindenden Doppelbindung der Akzeptorgruppe ist aus den Kalkulationen mit PM3 für folgende Verbindungen zu sehen:

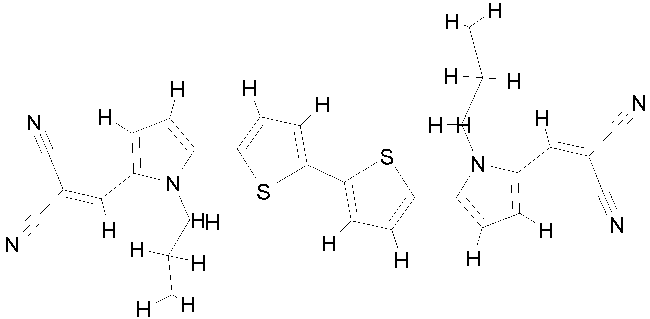

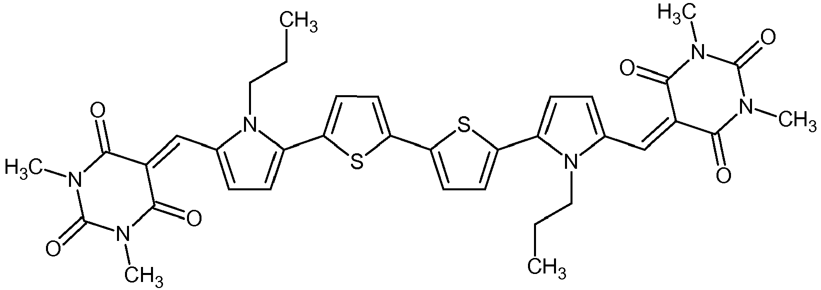

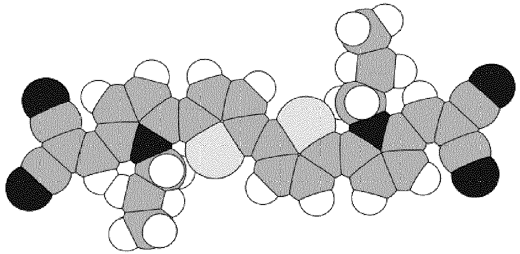

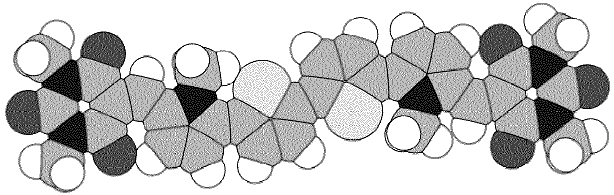

Die Herstellung der erfindungsgemäßen Verbindungen der allgemeinen Formel (I) lässt über vielfältige, dem Fachmann bekannte Reaktionsschritte realisieren. Ein kondensierter Baustein D aus drei aromatischen 5-Ringen und Y1=S, W1=C und Z1=NR lässt sich beispielsweise Zotti (Zotti et al, Makromol. Chem. 1992, 193, 399)

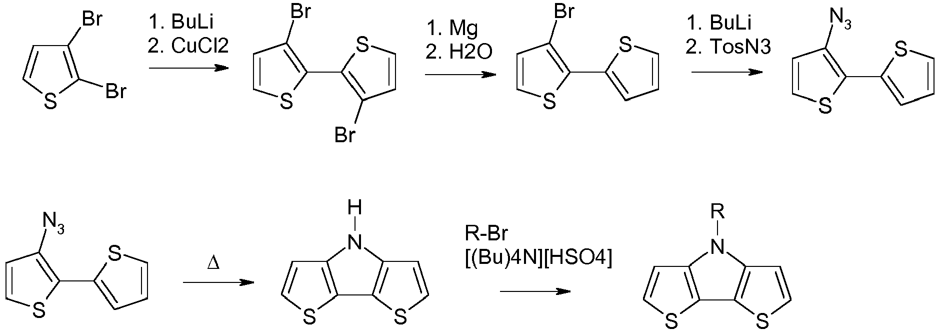

Oder nach Rasmussen (Rasmussen et al, Org. Chem. 2001, 66, 9067)

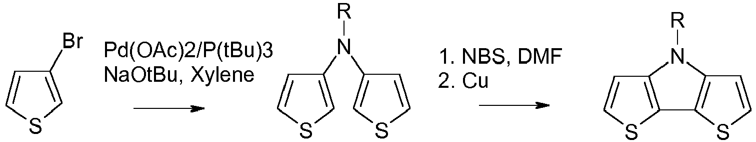

Oder nach Barlow (Barlow et al, Chem. Eur. J. 2007, 13, 9637) 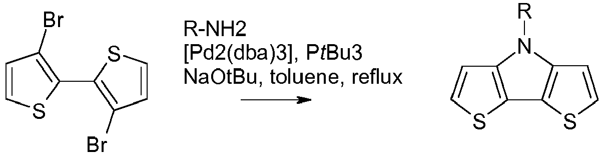 herstellen.

Ein kondensierter Baustein D aus drei aromatischen 5-Ringen und Y1=S und Z1=Si(RR`) lässt sich beispielsweise nach Hakan Usta, Gang Lu, Antonio Facchetti, Tobin J. Marks, J. Am. Chem. Soc. 2006 (128) 9034-9035 herstellen:

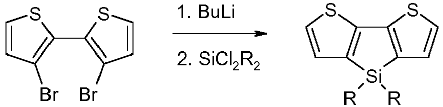

Die Herstellung der DCV-Pyrrol-Kopplungspartner erfolgt nach Literaturbekannten Methoden, z.B. Joshua A. Davies et al., J. Am. Chem. Soc. 2008 (130) 10565-10575 bis zur Aldehydstufe. Die Knoevenagelreaktion mit Malodinitril erfolgt nach der Vorschrift von M. Manuela M. Raposo et al., Org. Lett. 2006 (8) 3681-3684:

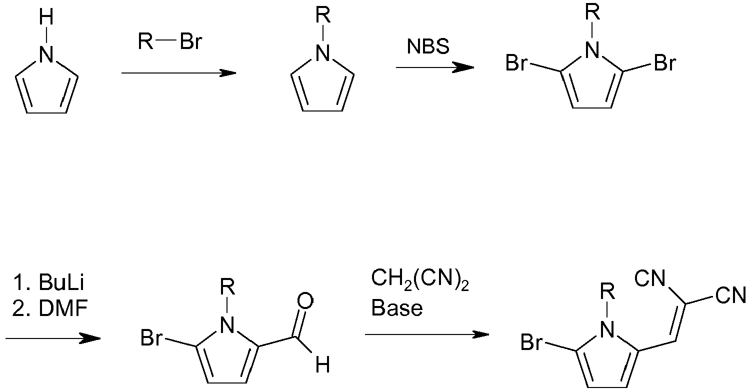

Eine inverse Reaktionsführung ist ebenfalls möglich, die Herstellung der benötigten Bausteine erfolgt nach der Literatur von Franck Denat, Hafida Gaspard-Iloughmane, Jacques Dubac, J. Organomet. Chem. 1992 (423) 173-182. Auch in diesem Fall wird die Knoevenagelreaktion nach der Vorschrift von M. Manuela M. Raposo et al. durchgeführt:

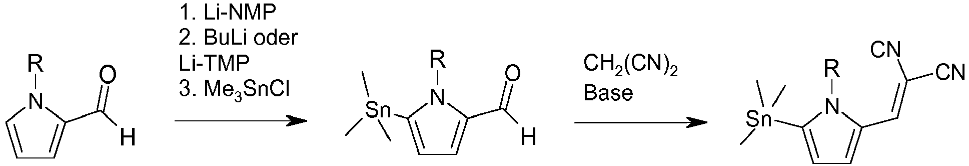

Die Kopplung des Bausteins D mit den beiden flankierenden Pyrrolen lässt sich über eine der üblichen Methoden zur unsymmetrischen Verknüpfung zweier (hetero-)Aromaten (Stille oder Negishi) durchführen. Dabei sind drei mögliche Varianten bevorzugt.

### 1.Variante, Stille-Reaktion:

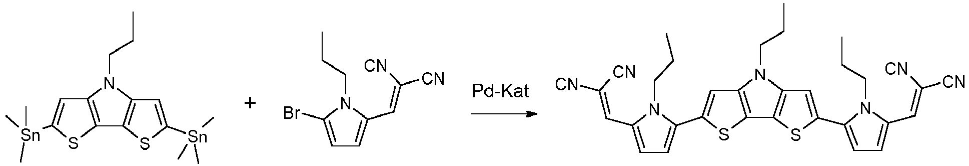

Ein Distannylierter Mittelblock D, wie in dem Reaktionsschema das abgebildete 4-Propyl-2,6-bis-trimethylstannyl-4H-dithieno[3,2-b;2',3'-d]pyrrol, wird mit dem bromierten Pyrrol-Baustein mittels Palladium-Katalyse gekoppelt.

### 2. Variante, inverse Stille-Reaktion:

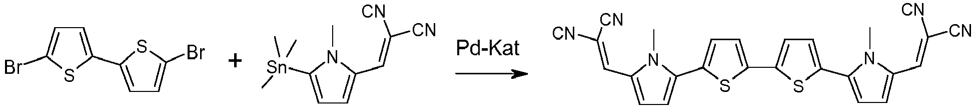

Ein Dibromierter Mittelblock D, wie in dem Reaktionsschema das abgebildete 5,5'-Dibrom-[2,2']bithiophenyl, wird mit dem stannylierten Pyrrol-Baustein mittels Palladium-Katalyse gekoppelt.

### 3. Variante, Negishi-Reaktion:

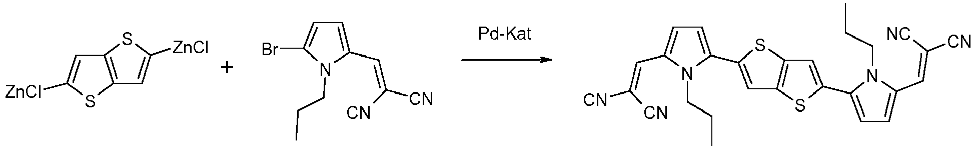

Ein Dizinkchlorid Mittelblock D, wie in dem Reaktionsschema das abgebildete 2,5-Dizinkchlorid-thieno[3,2-b]thiophen, wird mit dem bromierten Pyrrol-Baustein mittels Palladium-Katalyse gekoppelt.

Ebenso Gegenstand der Erfindung ist die Verwendung einer erfindungsgemäßen Verbindung nach der allgemeinen Formel (I) in einem optoelektronischen Bauelement.

Ebenso Gegenstand der Erfindung ist ein optoelektronisches Bauelement umfassend eine Elektrode und eine Gegenelektrode und zumindestens eine organische Schicht zwischen Elektrode und Gegenelektrode enthaltend eine Verbindung der allgemeinen Formel (I).

In einer Ausführungsform der Erfindung ist das optoelektronische Bauelement eine Solarzelle, eine lichtemittierende Diode (LED), ein Feldeffekttransistor (FET) oder Fotodetektor, wobei zumindestens eine Schicht eine erfindungsgemäße Verbindung nach allgemeiner Formel (I) umfasst.

In einer weiteren Ausführungsform der Erfindung ist das optoelektronische Bauelement eine organische Solarzelle oder eine Hybrid-Solarzelle aus organischen und anorganischen Materialien, wobei zumindestens eine erfindungsgemäße Verbindung nach allgemeiner Formel (I) vorhanden ist.

In einer weiteren Ausführungsform der Erfindung ist das optoelektronische Bauelement auf einem flexibel ausgeführten Substrat ausgeführt.

Unter einem flexiblen Substrat wird im Sinne der vorliegenden Erfindung ein Substrat verstanden, welches eine Verformbarkeit infolge äußerer Krafteinwirkung gewährleistet. Dadurch sind solche flexiblen Substrate zur Anordnung auf gekrümmten Oberflächen geeignet. Flexible Substrate sind beispielsweise Folien, textile Membrane oder Metallbänder.

In einer weiteren Ausführungsform der Erfindung ist das optoelektronische Bauelement als organische pin-Solarzelle bzw. organische pin-Tandemsolarzelle oder Mehrfachsolarzelle ausgeführt.

Als Tandemsolarzelle wird dabei eine Solarzelle bezeichnet, die aus einem vertikalen Stapel zweier in Serie verschalteter Solarzellen besteht. Als Mehrfachsolarzelle wird dabei eine Solarzelle bezeichnet, die aus einem vertikalen Stapel mehrerer in Serie verschalteter Solarzellen besteht, wobei maximal 10 Solarzellen in einem Stapel verschaltet sind.

In einer weiteren Ausführungsform der Erfindung ist das optoelektronische Bauelement eine Solarzelle, wobei das Absorbersystem eine erfindungsgemäße Verbindung der allgemeinen Formel (I) sowie einen weiteren Absorber, der bei Licht >600nm absorbiert, umfasst.

In einer weiteren Ausführungsform der Erfindung ist das optoelektronische Bauelement eine Tandemzelle mit zwei Absorbersystemen, wobei das eine Absorbersystem eine erfindungsgemäße Verbindung der allgemeinen Formel (I) umfasst und das andere Absorbersystem ein Absorbermaterial umfasst, dessen Absorptionsmaximum bei einer Wellenlänge > 600nm liegt.

In einer weiteren Ausführungsform der Erfindung sind in dem Bauelement noch eine oder mehrere undotierte, teilweise dotierte oder ganz dotierte Transportschichten vorhanden. Bevorzugt haben diese Transportschichten ein Maximum der Absorption bei < 450nm, besonders bevorzugt < 400nm.

In einer weiteren Ausführungsform der Erfindung sind die Schichten des Schichtsystems des Bauelements als eine den optischen Weg des einfallenden Lichts verlängernde Lichtfalle ausgebildet.

In einer weiteren Ausführungsform der Erfindung enthält mindestens eine der photoaktiven Mischschichten als Akzeptor ein Material aus der Gruppe der Fullerene bzw. Fullerenderivate (C60, C70, etc.), Subphthalocyanine, Rylene, Fluorene, Carbazole, Benzothiadiazole, Diketopyrrolopyrrole oder Vinazene.

In einer weiteren Ausführungsform der Erfindung bestehen die Kontakte aus Metall, einem leitfähigen Oxid, insbesondere ITO, ZnO:Al oder anderen TCOs, aus einer gestapelten Elektrode umfassend mehrere Schichten aus dünnem Metall oder dotierten oder undotierten Halbleitermaterialien, sogenannte DMD's, aus einer Elektrode umfassend Silber-Nanodrähte oder Kohlenstoffröhren oder andere Nanopartikel, die eine leitfähige Elektrodenschicht bilden oder einem leitfähigen Polymer, insbesondere PEDOT:PSS oder PANI.

In einer weiteren Ausführungsform ist zwischen der ersten elektronenleitenden Schicht (n-Schicht) und der auf dem Substrat befindlichen Elektrode noch eine p-dotierte Schicht vorhanden, so dass es sich um eine pnip oder pni-Struktur handelt, wobei vorzugsweise die Dotierung so hoch gewählt ist, dass der direkte pn-Kontakt keine sperrende Wirkung hat, sondern es zu verlustarmer Rekombination, bevorzugt durch einen Tunnelprozess kommt.

In einer weiteren Ausführungsform der oben beschriebenen Strukturen sind diese als organische Tandemsolarzelle oder Mehrfachsolarzelle ausgeführt. So kann es sich bei dem Bauelement um eine Tandemzelle aus einer Kombination aus nip, ni, ip, pnip, pni, pip, nipn, nin, ipn, pnipn, pnin oder pipn-Strukturen handeln, bei der mehrere unabhängige Kombinationen, die mindestens eine i-Schicht enthalten, übereinander gestapelt sind (Kreuzkombinationen).

In einer weiteren Ausführungsform der oben beschriebenen Strukturen ist diese als eine pnipnipn-Tandemzelle ausgeführt.

In einer Ausführungsform der Erfindung ist das Bauelement mit mindestens einer anorganischen Schicht beinhaltend eine oder mehrere anorganische Materialien aufgebaut.

In einer weiteren Ausführungsform der Erfindung wird das Bauelement auf ebenen, gekrümmten oder flexiblen Trägerflächen verwendet. Bevorzugt sind diese Trägerflächen Plastikfolien oder Metallfolien (z.B. Aluminium, Stahl), etc.

Nachfolgend soll die Erfindung anhand einiger Ausführungsbeispiele und dazugehöriger Figuren eingehender erläutert werden. Es zeigen die
Fig.1 eine Reaktionsschema zur Herstellung der erfindungsgemäßen Verbindung 1, in
Fig.2 eine schematische Darstellung des Absorptionsspektrums von Verbindung 1 im Vergleich zu bauähnlichen nicht erfindungsgemäßen Verbindungen, in
Fig.3 eine schematische Darstellung des Absorptionsspektrums von Verbindung 2 im Vergleich zu bauähnlichen nicht erfindungsgemäßen Verbindungen, in
Fig.4 eine schematische Darstellung des Absorptionsspektrums von Verbindung 3 im Vergleich zu bauähnlichen nicht erfindungsgemäßen Verbindungen, in
Fig.5 eine schematische Darstellung einer Strom-SpannungsKurve einer MIP-Zelle mit Schicht von Verbindung 1, in
Fig.6 eine schematische Darstellung einer Strom-SpannungsKurve einer MIP-Zelle mit Schicht von Verbindung 2, in
Fig.7 eine schematische Darstellung einer Strom-SpannungsKurve einer MIP-Zelle mit Schicht von Verbindung 3, in
Fig.8 eine schematische Darstellung einer Strom-SpannungsKurve einer MIP-Zelle mit Schicht von Verbindung 4, in

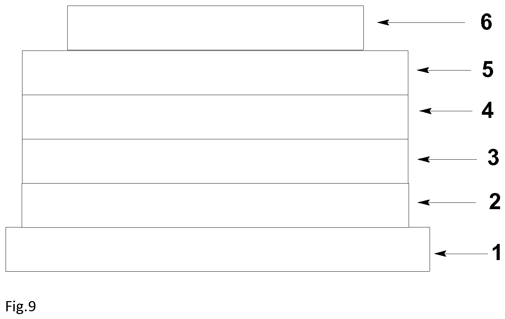

Figur 9 die schematische Darstellung einer Struktur eines beispielhaften photoaktiven Bauelements, wobei 1 das Substrat ist, 2 eine erste Elektrode z.B. ITO, 3 eine dotierte oder undotierte Ladungstransportschicht, z.B. eine Elektronentransportschicht (ETL), 4 eine organische photoaktive Schicht, 5 eine dotierte oder undotierte Ladungstransportschicht, z.B. eine Löchertransportschicht (HTL) und 6 eine zweite Elektrode, z.B. aus Gold ist.

In den aufgeführten Ausführungsbeispielen sind beispielhaft einige erfindungsgemäßen Bauelemente aufgezeigt. Zur Charakterisierung wichtige Kennzahlen sind der Füllfaktor, die Leerlaufspannung und Kurzschlussstrom aufgelistet, die aus der Strom-Spannungs-Kennlinie entnommen werden. Die Ausführungsbeispiele sollen die Erfindung beschreiben ohne sich auf diese zu beschränken.

### Ausführungsbeispiel 1: Herstellung von Verbindung 1

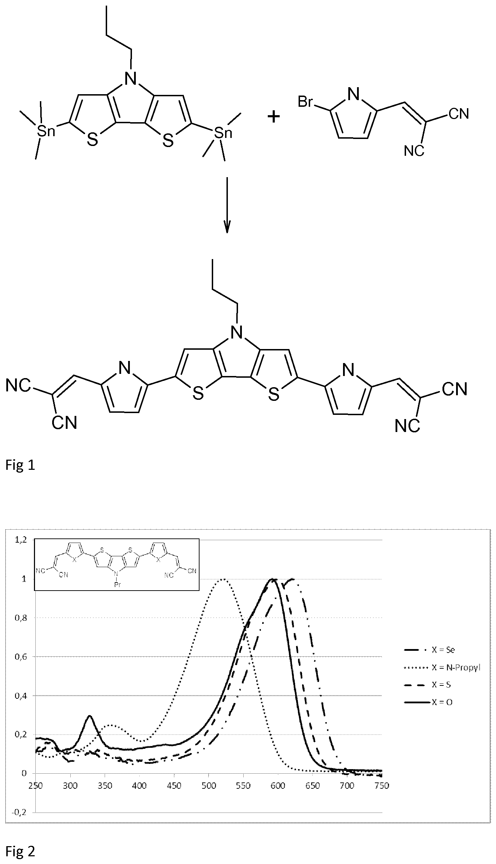

In einem ersten Ausführungsbeispiel ist in Fig. 1 schematisch die Darstellung der Verbindungen 1 gezeigt

Die Synthesen werden in der Regel nach der allgemeinen Arbeitsvorschrift 1 (AAV 1) durchgeführt:
Die Bisstannyl-Verbindung und 2,5 bis 3,0 äquivalente der Brom-Pyrrol-Verbindung werden in trockenem THF oder Toluol gelöst, bei inverser Reaktionsführung verwendet man die entsprechende Dibrom-Verbindung und 2,5 bis 3,0 äquivalente der Stannyl-Pyrrol-Verbindung. Pd(PPh₃)₄ oder Pd(*tert*-Bu)₂ wird hinzugegeben und die Reaktionsmischung wird erhitzt. Nach dem Abkühlen auf Raumtemperatur wird filtriert und der Rückstand wird mit Methanol gewaschen. Das Rohprodukt wird durch umkristallisieren gereinigt.

Nach AAV1, 2-(5-Brom-1-propyl-1H-pyrrol-2-ylmethylene)-malonitril (1030 mg, 3,90 mmol) und 4-Propyl-2,6-bistrimethylstannyl-4H-dithieno[3,2-b;2',3'-d]pyrrol (822 mg, 1,50 mmol) werden in 10 ml THF gelöst, Pd(PPh₃)₄ (18 mg, 0,02 mmol) wird hinzugegeben und die Mischung wird für 48 h auf 70°C erhitzt. Nach der Aufarbeitung wird das Rohprodukt aus Chlorbenzol umkristallisiert und man erhält 530 mg (60%) Produkt. ¹H-NMR (TCE-d2, 375 K): 7,71 ppm (d, 2H), 7,39 (s, 2H), 7,10 (s, 2H), 6,65 (d, 2H), 4,16 (m, 6H), 1,92 (m, 2H), 1,76 (m, 4H), 0,97 (t, 3H), 0,93 (t, 6H). UV (Film): 576 nm.

Fig. 2 zeigt einen Vergleich der Absorptionsmaxima der erfindungsgemäßen Verbindung 1 im Vergleich zu Verbindungen bei denen die Pyrrolringe durch andere heterocyclische 5-Ringe wie Furan, Thiophen oder Selenophen ersetzt sind. Man sieht für die erfindungsgemäße Verbindung 1 eine deutliche Verschiebung des Absorptionsmaximum zu kürzeren Wellenlängen.

### Ausführungsbeispiel 2: Herstellung von Verbindung 2

Nach AAV1, 2-(5-Brom-1-propyl-1H-pyrrol-2-ylmethylene)-malonitril (687 mg, 2,60 mmol) und 5,5'-Bis-trimethylstannyl-[2,2']bithiophenyl (492 mg, 1,00 mmol) werden in 7 ml THF gelöst, Pd(PPh₃)₄ (12 mg, 0,01 mmol) wird hinzugegeben und die Mischung wird für 48 h auf 70°C erhitzt. Nach der Aufarbeitung wird das Rohprodukt aus Chlorbenzol umkristallisiert und man erhält 170 mg (32%) Produkt. ¹H-NMR (TCE-d2, 375 K): 7,69 ppm (d, 2H), 7,39 (s, 2H), 7,23 (d, 2H), 7,11 (d, 2H), 6,61 (d, 2H) 4,10 (dd, 2H), 1,73 (m, 4H), 0,92 (t, 6H). UV (Film): 506 nm.

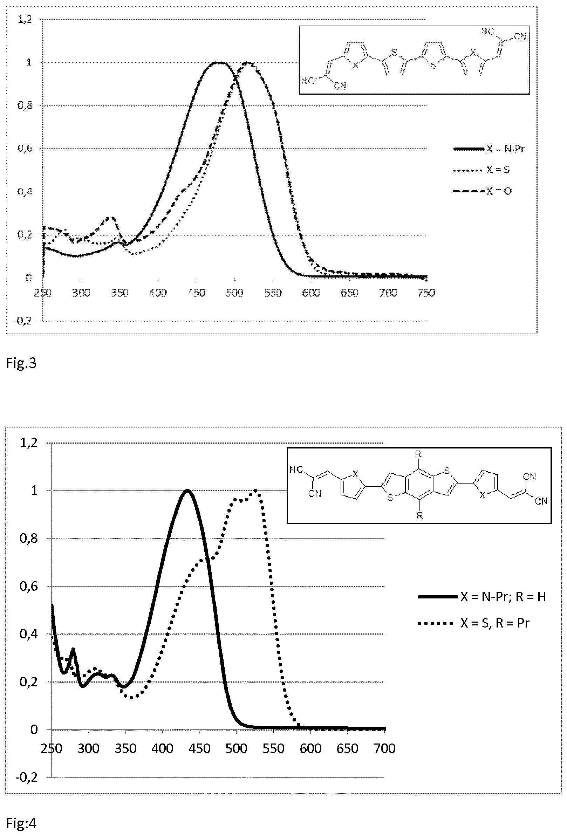

Fig 3 zeigt einen Vergleich der Absorptionsmaxima der erfindungsgemäßen Verbindung 2 im Vergleich zu Verbindungen bei denen die Pyrrolringe durch andere heterocyclische 5-Ringe wie Furan oder Thiophen ersetzt sind. Man sieht für die erfindungsgemäße Verbindung 2 eine deutliche Verschiebung des Absorptionsmaximums zu kürzeren Wellenlängen.

### Ausführungsbeispiel 3: Herstellung von Verbindung 3

### Herstellung von Sn-TPhT

In einem mit Argon belüfteten 100 ml 3-Halsrundkolben wird benzo[1,2-b;4,5-b']dithiophen (TPhT) (400 mg, 2.1 mmol) in 50 ml THF (abs.) gelöst und auf -78°C gekühlt. tBuLi (1.6 M in Hexan, 2.65 ml, 4.25 mmol) wird zugetropft, das Reaktionsgemisch wird ca.1,5 Stunden bei -78°C gehalten. Me₃SnCl (1.0 M in Hexan, 4.25 ml, 4.25 mmol) wird zugegeben und nach ca. 1 Stunde wird die Reaktionsmischung aufgetaut. Nach 2 Stunden wird Wasser (50 ml) zugegeben, die wässrige Phase wird mit 2X50 ml Et20 extrahiert. Die vereinigten organischen Phasen werden getrocknet und eingeengt. Ausbeute 930 mg (86%) von (Sn-TPhT). NMR ¹H: (CDCl₃) 8.27 (s, 2H), 7.43 (s, 2H), 0.44 (s,18H)

### Herstellung von Verbindung 3

Nach allgemeine Arbeitsvorschrift 1 oder: In einem Reaktionsgefäß werden unter Argon (Sn-TPhT) (890 mg, 1.72 mmol), 2-(5-Brom-1-propyl-1H-pyrrol-2-ylmethylydene)-malononitril (1.09 g, 4.12 mmol) und 150 mg Pd[PPh₃]₄ vorgelegt, 35 ml Toluol zugegeben und 24 Stunden unter Rückfluss erhitzt. Der Niederschlag wird getrennt und 2 mal kristallisiert (PhCl). Ausbeute: 450 mg (47 %) von Verbindung 3.

Fig. 4 zeigt einen Vergleich der Absorptionsmaxima der erfindungsgemäßen Verbindung 3 im Vergleich zu Verbindungen bei denen die Pyrrolringe durch Thiophene ersetzt sind. Man sieht für die erfindungsgemäße Verbindung 3 eine deutliche Verschiebung des Absorptionsmaximums zu kürzeren Wellenlängen.

### Ausführungsbeispiel 4: Herstellung von Verbindung 4

357 mg (2,55 mmol) Thienothiophen in 20 ml trock. THF lösen und auf -78 °C abkühlen. 3,2 ml (5,10 mmol) 1,6-M-Butyllithium langsam zu geben und 1 h bei -78 °C rühren. 5,1 ml (5,10 mmol) 1-M-Zinkchloridlösung zu geben und 2 h bei Raumtemperatur rühren. Anschließend, 1,35 g (5,10 mmol) 2-(5-Bromo-1-propyl-1H-pyrrol-2-ylmethylene)-malononitrile und 304 mg (0,26 mmol) Tetrakis(triphenylphosphine)palladium(0) dazu geben und das Reaktionsgemisch zum Sieden erhitzen. Nach 24h auf Raumtemperatur kommen lassen. Das Gemisch filtrieren, Rückstand mit THF und Methanol waschen. Das Rohprodukt aus Chlorbenzol umkristallisieren. Weitere Aufreinigung erfolgt durch Sublimation. Man erhält 60 mg (5%) gewünschtes Produkt. Smp (DSC) 371 °C. ¹H-NMR (TCE-d2, 80 °C), ppm: 7,83 (d, 1H), 7,54 (s, 1H), 7,46 (s, 1H), 6,77 (d, 1H), 4,25 (t, 2H), 1,87 (qa, 2H), 1,03 (t, 3H). UV (Film): 502 nm.

Das Absorptionsmaximum von erfindungsgemäßen Verbindung 4 in Lösung liegt bei 468 nm. Das Absorptionsmaximum der käuflichen Verbindung mit Thiophenen an Stelle der Pyrrole liegt in Lösung bei 510nm

### Ausführungsbeispiel 5: Bauelement mit der erfindungsgemäßen Verbindung 1:

In einem weiteren Ausführungsbeispiel wird ein MIP-Bauelement bestehend aus einer Probe auf Glas mit transparentem Deckkontakt ITO, einer Schicht Buckminster Fulleren C₆₀, einer Schicht der erfindungsgemäßen Verbindung 1, einer dotierten Löchertransportschicht, und einer Schicht Gold hergestellt.

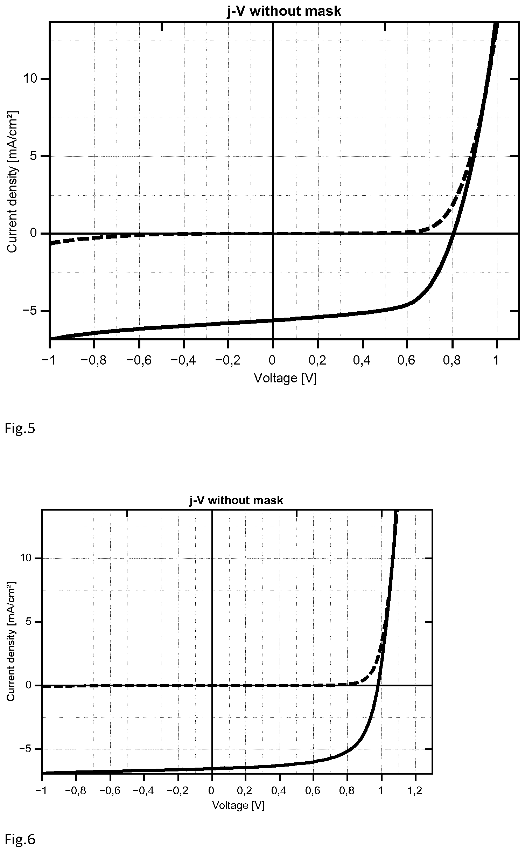

Fig. 5 zeigt die Strom-Spannungskurve dieser MIP-Zelle. Die gestrichelte Kurve zeigt die Dunkelkennlinie.

Die wichtigsten Kennzahlen wie der Füllfaktor FF mit 61.2%, die Leerlaufspannung U_{oc} mit 0.81V und der Kurzschlussstrom Isc mit 6.6 mA zeigen eine gut funktionierende organische Solarzelle.

### Ausführungsbeispiel 6: Bauelement mit der erfindungsgemäßen Verbindung 2:

In einem weiteren Ausführungsbeispiel wird ein MIP-Bauelement bestehend aus einer Probe auf Glas mit transparentem Deckkontakt ITO, einer Schicht Buckminster Fulleren C₆₀, einer Schicht der erfindungsgemäßen Verbindung 2, einer dotierten Löchertransportschicht, und einer Schicht Gold hergestellt.

Fig.6 zeigt die Strom-Spannungskurve dieser MIP-Zelle. Die gestrichelte Kurve zeigt die Dunkelkennlinie.

Die wichtigsten Kennzahlen wie der Füllfaktor FF mit 64.9%, die Leerlaufspannung U_{oc} mit 0.98V und der Kurzschlussstrom Isc mit 7.1 mA zeigen eine gut funktionierende organische Solarzelle.

### Ausführungsbeispiel 7: Bauelement mit der erfindungsgemäßen Verbindung 3:

In einem weiteren Ausführungsbeispiel wird ein MIP-Bauelement bestehend aus einer Probe auf Glas mit transparentem Deckkontakt ITO, einer Schicht Buckminster Fulleren C₆₀, einer Schicht der erfindungsgemäßen Verbindung 3, einer dotierten Löchertransportschicht, und einer Schicht Gold hergestellt.

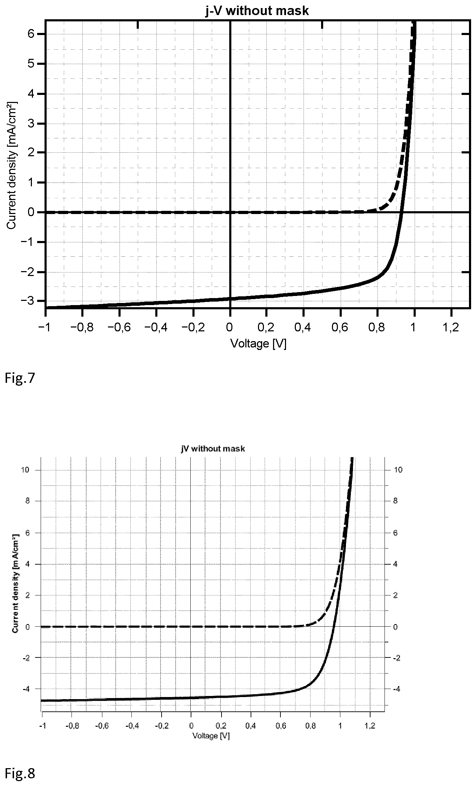

Fig. 7 zeigt die Strom-Spannungskurve dieser MIP-Zelle. Die gestrichelte Kurve zeigt die Dunkelkennlinie.

Die wichtigsten Kennzahlen wie der Füllfaktor FF mit 65.1%, die Leerlaufspannung U_{oc} mit 0.93V und der Kurzschlussstrom Isc mit 3.0 mA zeigen eine gut funktionierende organische Solarzelle.

### Ausführungsbeispiel 8: Bauelement mit der erfindungsgemäßen Verbindung 4:

In einem weiteren Ausführungsbeispiel wird ein MIP-Bauelement bestehend aus einer Probe auf Glas mit transparentem Deckkontakt ITO, einer Schicht Buckminster Fulleren C₆₀, einer Schicht der erfindungsgemäßen Verbindung 4, einer dotierten Löchertransportschicht, und einer Schicht Gold hergestellt.

Fig.8 zeigt die Strom-Spannungskurve dieser MIP-Zelle. Die gestrichelte Kurve zeigt die Dunkelkennlinie.

Die wichtigsten Kennzahlen wie der Füllfaktor FF mit 68.2%, die Leerlaufspannung U_{oc} mit 0.96V und der Kurzschlussstrom Isc mit 5.3 mA zeigen eine gut funktionierende organische Solarzelle.

### Bezugszeichenliste

- 1: Substrat
- 2: Elektrode
- 3: Transportschichtsystem (ETL bzw. HTL)
- 4: organisches lichtempfindliches Schichtsystem
- 5: Transportschichtsystem (ETL bzw. HTL
- 6: Gegenelektrode

## Patentansprüche

1. Optoelektronisches Bauelement umfassend eine Elektrode (2) und eine Gegenelektrode (6) und mindestens eine organische Schicht (4) zwischen der Elektrode (2) und der Gegenelektrode (6), **dadurch gekennzeichnet, dass** die mindestens eine organische Schicht (4) eine Verbindung der allgemeinen Formel(I) 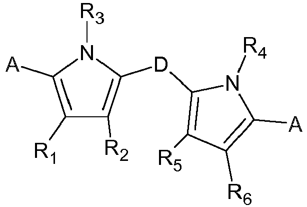
a. umfasst, mit R₃ und R₄ unabhängig ausgesucht aus Alkyl, Aryl oder substituiertes Alkyl mit 1-26 C-Atomen, wobei maximal 2 C-Atome ersetzt sind durch N, O oder S oder maximal 5 H-Atome ersetzt sind durch F, Cl, Br oder CN,
b. R₁, R₂, R₅ und R₆ unabhängig voneinander ausgesucht H, Alkyl oder O-Alkyl, wobei R₁ und R₂ und/oder R₅ und R₆ einen Ring bilden können,
c. und wobei A ausgesucht ist aus: 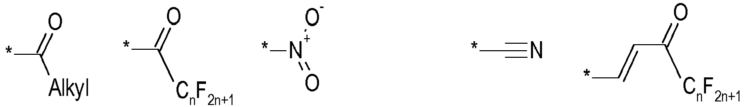 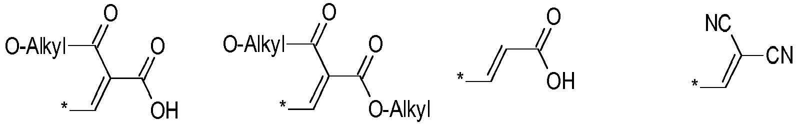 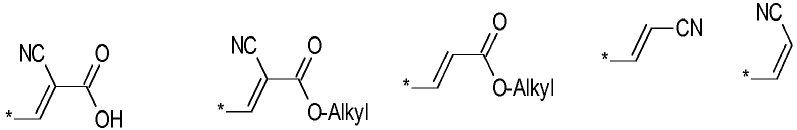 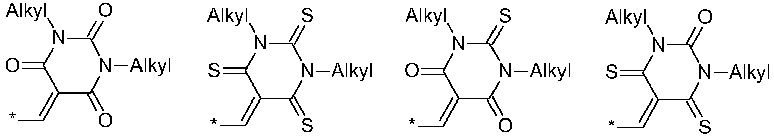 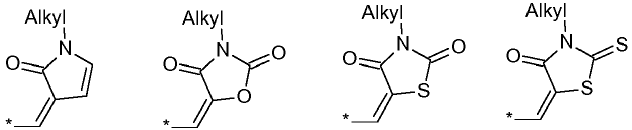 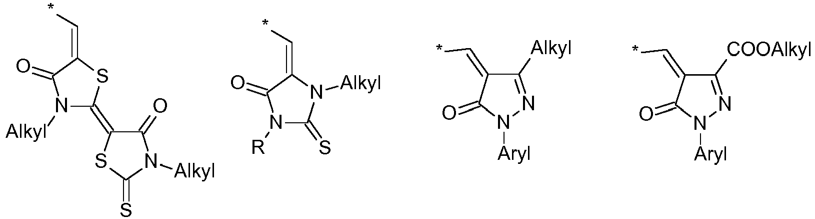 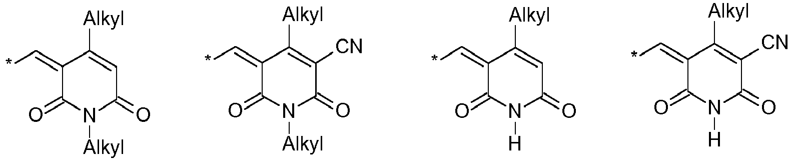 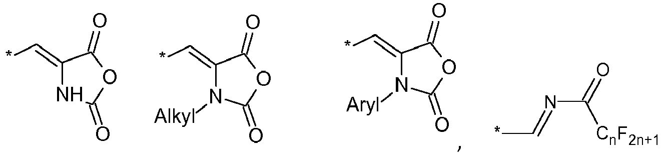 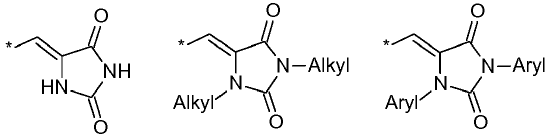
d. wobei n eine ganze Zahl von 1 bis 5 ist,
e. und wobei D eine Einheit aus linear konjugierten aromatischen 5- und/oder 6-Ringen ist, die anneliert sein können,
f. wobei D ausgesucht ist aus: 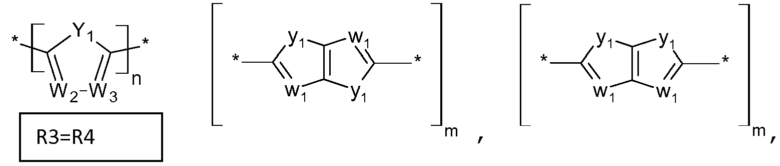 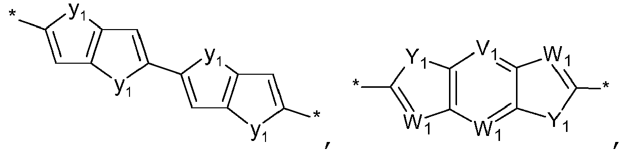 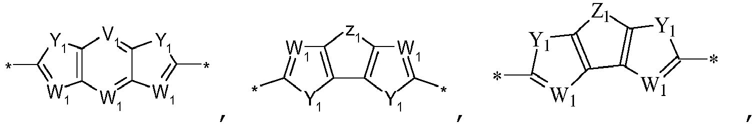 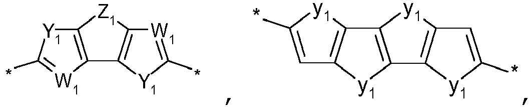 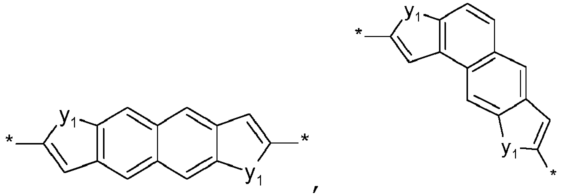
wobei Y₁ und Z₁ unabhängig ausgewählt sind aus: O, S, Se, Si(RR'), C(RR') und N(R), mit R und R' unabhängig voneinander ausgewählt aus H, Alkyl oder Aryl und
W₁ und V₁ unabhängig ausgewählt ist aus: N und C-R`, mit R` unabhängig voneinander ausgewählt aus H, Alkyl, O-Alkyl, S-Alkyl oder Aryl
W₂ jeweils unabhängig ausgewählt ist aus: N und C-R9 mit R9 = H, Halogen, Alkyl, Alkenyl, Alkinyl, jeweils linear oder verzweigt, OR', SR`, SiR'3, NR'2 mit R` ausgesucht aus Alkyl oder Aryl,
W₃ jeweils unabhängig ausgewählt ist aus: N und C-R10 mit R10 = H, Halogen, Alkyl, Alkenyl, Alkinyl, jeweils linear oder verzweigt, OR', SR`, SiR'3, NR'2 mit R' ausgesucht aus Alkyl oder Aryl,
wobei R9 und R10 einen Ring bilden können, und
n = 1, 2 oder 3 und m = 1 oder 2 ist.

2. Optoelektronisches Bauelement nach Anspruch 1, wobei A ausgesucht ist aus: 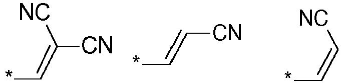 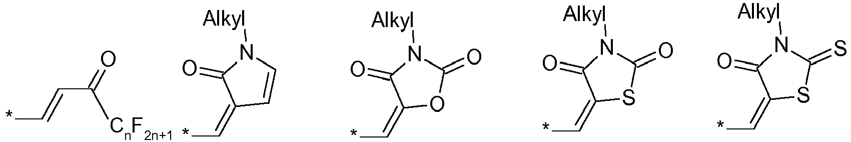 wobei n eine ganze Zahl von 1 bis 5 ist.

3. Optoelektronisches Bauelement nach Anspruch 1, wobei R₃=R₄=H ist und A ausgesucht aus

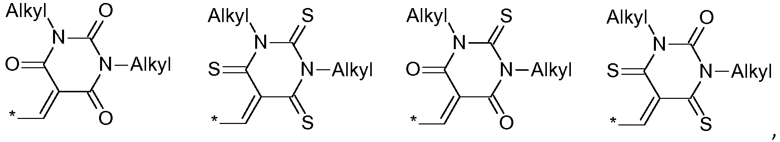

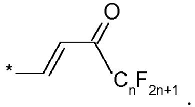

4. Optoelektronisches Bauelement nach Anspruch 1, wobei R3 und R4 unabhängig voneinander Alkyl, Aryl oder substituiertem Alkyl mit 1-26 C-Atomen, wobei maximal 2 C-Atome ersetzt sind durch N, O oder S oder maximal 5 H-Atome ersetzt sind durch F, Cl, Br oder CN und A ausgesucht aus

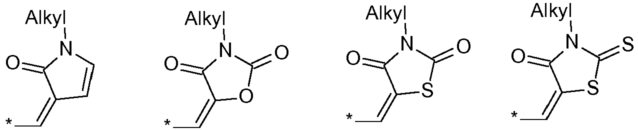

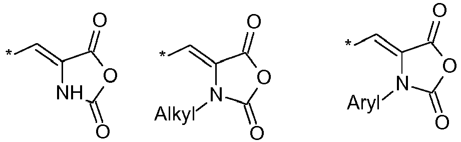

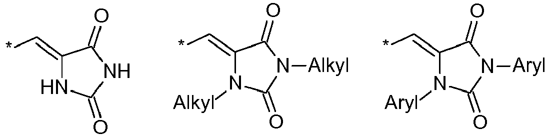

5. Optoelektronisches Bauelement nach Anspruch 1, wobei A jeweils gleich und ausgesucht aus cis-CH=CHCN, trans-CH=CHCN oder CH=C(CN)₂ ist.

6. Optoelektronisches Bauelement nach einem der Ansprüche 1 bis 5, wobei D ausgesucht ist aus: 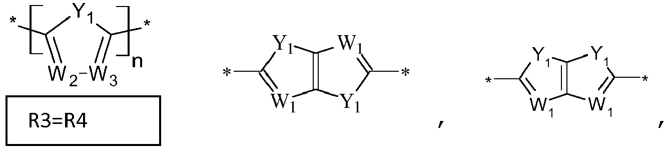 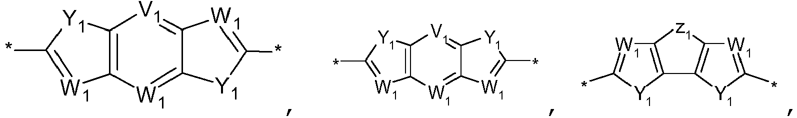 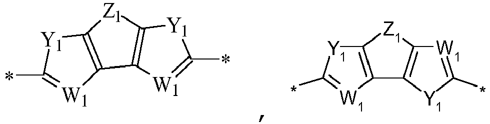
wobei Y₁ und Z₁ unabhängig ausgewählt sind aus: O, S, Se, Si(RR'), C(RR') und N(R), mit R und R' unabhängig voneinander ausgewählt aus H, Alkyl oder Aryl und
W₁ und V₁ unabhängig ausgewählt ist aus: N und C-R`, mit R` unabhängig voneinander ausgewählt aus H, Alkyl, O-Alkyl, S-Alkyl oder Aryl
W₂ jeweils unabhängig ausgewählt ist aus: N und C-R9 mit R9 = H, Halogen, Alkyl, Alkenyl, Alkinyl, jeweils linear oder verzweigt, OR', SR`, SiR'3, NR'2 mit R` ausgesucht aus Alkyl oder Aryl,
W₃ jeweils unabhängig ausgewählt ist aus: N und C-R10 mit R10 = H, Halogen, Alkyl, Alkenyl, Alkinyl, jeweils linear oder verzweigt, OR', SR`, SiR'3, NR'2 mit R' ausgesucht aus Alkyl oder Aryl,
wobei R9 und R10 einen Ring bilden können, und
n = 1, 2 oder 3 und m = 1 oder 2 ist.

7. Optoelektronisches Bauelement nach einem der Ansprüche 1 bis 6, wobei A jeweils gleich ist und ausgesucht aus cis-CH=CHCN, trans-CH=CHCN oder CH=C(CN)₂ ist und
wobei die Gruppe D ausgesucht ist aus: 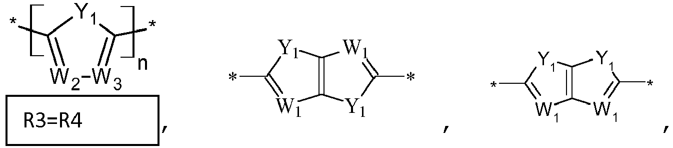 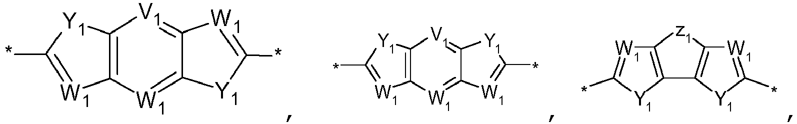 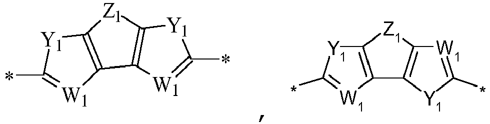
wobei Y₁ und Z₁ unabhängig ausgewählt ist aus O, S, Se, Si(RR'), C(RR') oder N(R), mit R und R' unabhängig voneinander ausgewählt aus H, Alkyl oder Aryl und
W₁ und V₁ unabhängig ausgewählt ist aus N oder C-R`, mit R' unabhängig voneinander ausgewählt aus H, Alkyl, O-Alkyl, S-Alkyl oder Aryl
W₂ jeweils unabhängig ausgewählt ist aus N oder C-R9 mit R9 unabhängig ausgewählt aus H, Halogen, Alkyl, Alkenyl, Alkinyl, jeweils linear oder verzweigt, OR', SR`, SiR'3, NR'2 mit R` ausgesucht aus Alkyl oder Aryl,
W₃ jeweils unabhängig ausgewählt ist aus N oder C-R10 mit R10 unabhängig ausgewählt aus H, Halogen, Alkyl, Alkenyl, Alkinyl, jeweils linear oder verzweigt, OR', SR', SiR'3, NR'2 mit R` ausgesucht aus Alkyl oder Aryl,
wobei R9 und R10 einen Ring bilden können, und
n = 1, 2 oder 3 ist.

8. Optoelektronisches Bauelement nach einem der Ansprüche 1 bis 7, wobei in der mindestens einen Verbindung der allgemeinen Formel (I) D ausgesucht ist aus 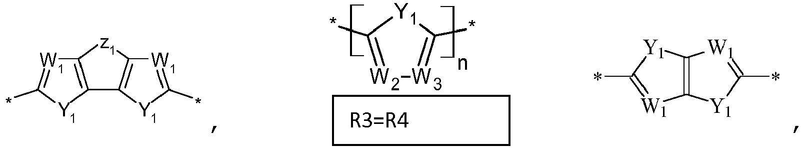 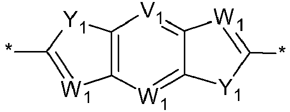
wobei Y₁ unabhängig ausgewählt ist aus O, S, Se oder N(R), mit R ausgewählt aus Alkyl oder Aryl,
Z₁ ausgewählt ist aus S, Se, Si(RR'), C(RR') oder N(R), mit R und R' unabhängig voneinander ausgewählt aus Alkyl oder Aryl,
W₁ und V₁ C-R ist, mit R unabhängig ausgewählt aus H, Alkyl oder Aryl,
W₂ unabhängig ausgewählt ist aus N oder C-R9 mit R9 unabhängig ausgewählt H, Halogen, Alkyl, Alkenyl, Alkinyl, jeweils linear oder verzweigt, OR', SR`, SiR'3, NR'2 mit R' ausgesucht aus Alkyl oder Aryl,
W3 unabhängig ausgewählt ist aus N und C-R10 mit R10 unabhängig ausgewählt aus H, Halogen, Alkyl, Alkenyl, Alkinyl, jeweils linear oder verzweigt, OR', SR`, SiR'3, NR'2 mit R' ausgesucht aus Alkyl oder Aryl,
wobei R9 und R10 einen Ring bilden können, und
n = 1, 2 oder 3 ist.

9. Optoelektronisches Bauelement nach einem der Ansprüche 1 bis 8, **dadurch gekennzeichnet, dass** in der mindestens einen Verbindung der allgemeinen Formel (I) R3 = R4 ist.

10. Optoelektronisches Bauelement nach einem der Ansprüche 1 bis 9, **dadurch gekennzeichnet, dass** es sich um eine organische Solarzelle oder eine Hybrid-Solarzelle handelt.

11. Optoelektronisches Bauelement nach einem der Ansprüche 1 bis 5, **dadurch gekennzeichnet, dass** es sich um eine organische Tandem-Solarzelle oder Mehrfachsolarzelle handelt.

12. Verbindungen der allgemeinen Formel(I) 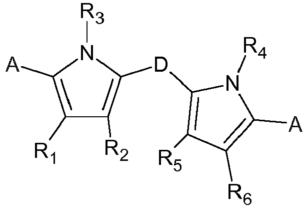
mit R₃ und R₄ unabhängig ausgesucht aus Alkyl, Aryl oder substituiertes Alkyl mit 1-26 C-Atomen, wobei maximal 2 C-Atome ersetzt sind durch N, O oder S oder maximal 5 H-Atome ersetzt sind durch F, Cl, Br oder CN,
R1, R2, R5 und R6 unabhängig voneinander ausgesucht H, Alkyl oder O-Alkyl, wobei R1 und R2 und/oder R5 und R6 einen Ring bilden können,
und wobei A ausgesucht ist aus: 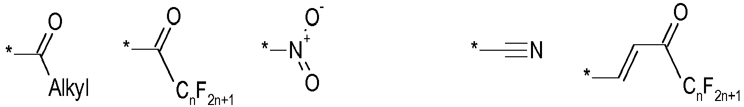 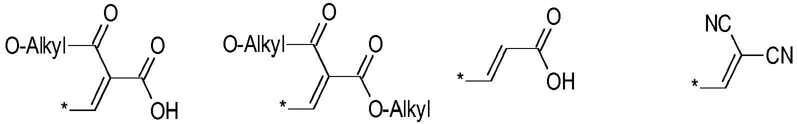 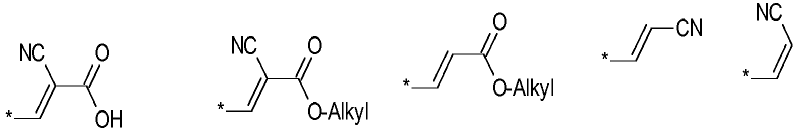 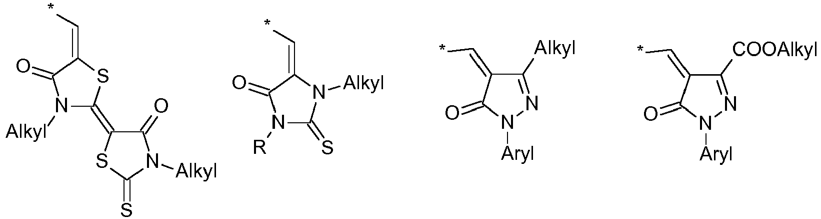 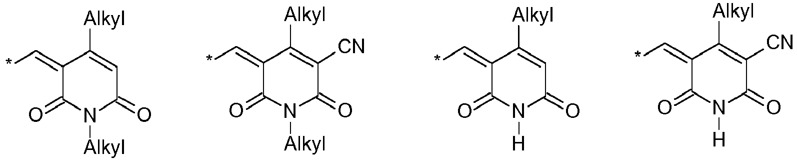 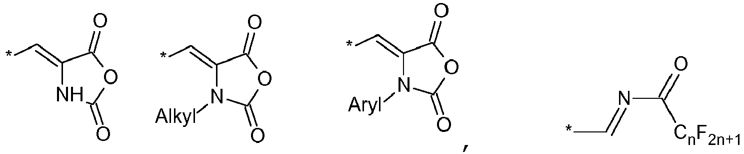 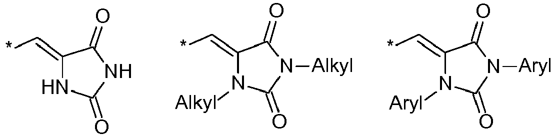
wobei n eine ganze Zahl von 1 bis 5 ist
und wobei D eine Einheit aus linear konjugierten aromatischen 5- und/oder 6-Ringen ist, die anneliert sein können,
wobei D ausgesucht ist aus: 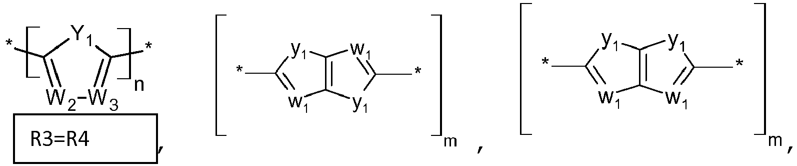 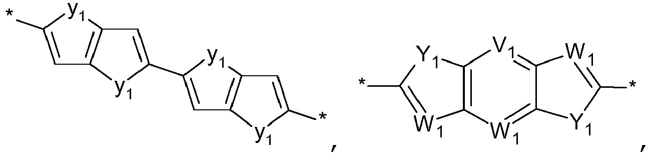 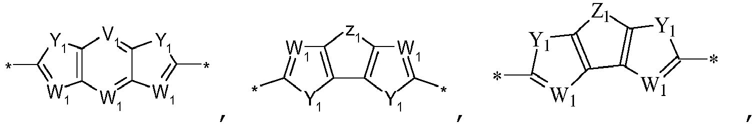 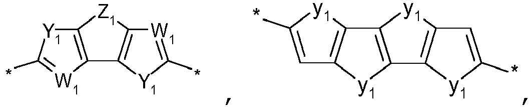 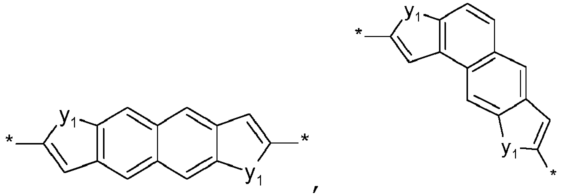
wobei Y₁ und Z₁ unabhängig ausgewählt sind aus: O, S, Se, Si(RR'), C(RR') und N(R), mit R und R' unabhängig voneinander ausgewählt aus H, Alkyl oder Aryl und
W₁ und V₁ unabhängig ausgewählt ist aus: N und C-R`, mit R` unabhängig voneinander ausgewählt aus H, Alkyl, O-Alkyl, S-Alkyl oder Aryl
W₂ jeweils unabhängig ausgewählt ist aus: N und C-R9 mit R9 = H, Halogen, Alkyl, Alkenyl, Alkinyl, jeweils linear oder verzweigt, OR', SR`, SiR'3, NR'2 mit R` ausgesucht aus Alkyl oder Aryl,
W₃ jeweils unabhängig ausgewählt ist aus: N und C-R10 mit R10 = H, Halogen, Alkyl, Alkenyl, Alkinyl, jeweils linear oder verzweigt, OR', SR', SiR'3, NR'2 mit R' ausgesucht aus Alkyl oder Aryl,
wobei R9 und R10 einen Ring bilden können, und
n = 1, 2 oder 3 und m = 1 oder 2 ist.

13. Verbindungen nach Anspruch 12, wobei A ausgesucht ist aus: 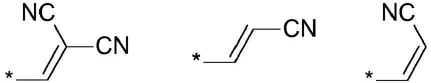 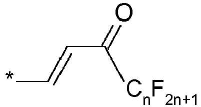 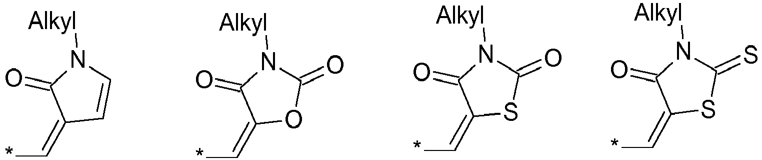 wobei n eine ganze Zahl von 1 bis 5 ist.

14. Verbindungen nach Anspruch 12, wobei R3=R4=H ist und A ausgesucht aus

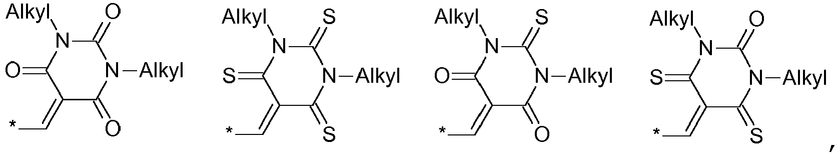

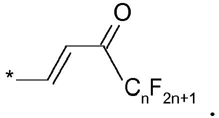

15. Verbindungen nach Anspruch 12, wobei R3 und R4 unabhängig voneinander Alkyl, Aryl oder substituiertem Alkyl mit 1-26 C-Atomen, wobei maximal 2 C-Atome ersetzt sind durch N, O oder S oder maximal 5 H-Atome ersetzt sind durch F, Cl, Br oder CN und A ausgesucht aus

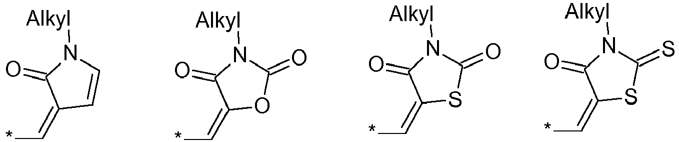

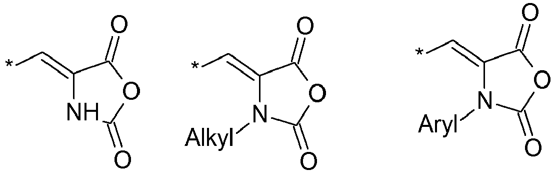

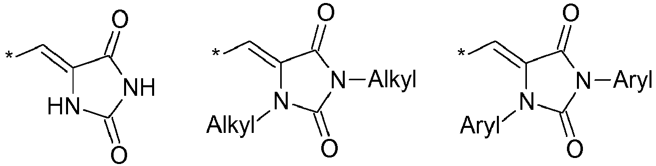

16. Verbindungen nach Anspruch 12, wobei A jeweils gleich und ausgesucht aus cis-CH=CHCN, trans-CH=CHCN oder CH=C(CN)₂ ist.

17. Verbindungen nach Anspruch 12 oder 16, wobei D ausgesucht ist aus: 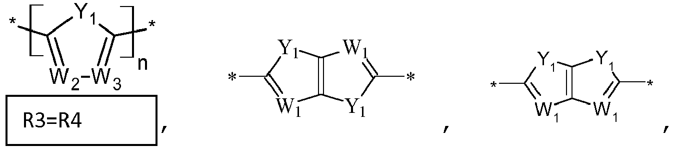 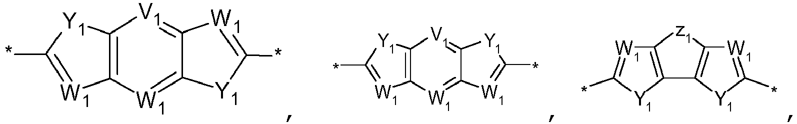 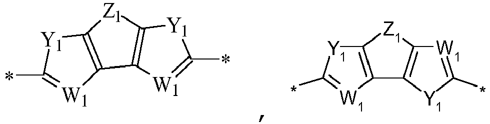
wobei Y₁ und Z₁ unabhängig ausgewählt sind aus: O, S, Se, Si(RR'), C(RR') und N(R), mit R und R' unabhängig voneinander ausgewählt aus H, Alkyl oder Aryl und
W₁ und V₁ unabhängig ausgewählt ist aus: N und C-R', mit R' unabhängig voneinander ausgewählt aus H, Alkyl, O-Alkyl, S-Alkyl oder Aryl
W₂ jeweils unabhängig ausgewählt ist aus: N und C-R9 mit R9 = H, Halogen, Alkyl, Alkenyl, Alkinyl, jeweils linear oder verzweigt, OR', SR', SiR'3, NR'2 mit R' ausgesucht aus Alkyl oder Aryl,
W₃ jeweils unabhängig ausgewählt ist aus: N und C-R10 mit R10 = H, Halogen, Alkyl, Alkenyl, Alkinyl, jeweils linear oder verzweigt, OR', SR', SiR'3, NR'2 mit R' ausgesucht aus Alkyl oder Aryl,
wobei R9 und R10 einen Ring bilden können, und
n = 1, 2 oder 3 und m = 1 oder 2 ist.

18. Verbindungen nach Anspruch 12, wobei A ist jeweils gleich und ausgesucht aus cis-CH=CHCN, trans-CH=CHCN oder CH=C(CN)₂ ist,
wobei die Gruppe D ausgesucht ist aus: 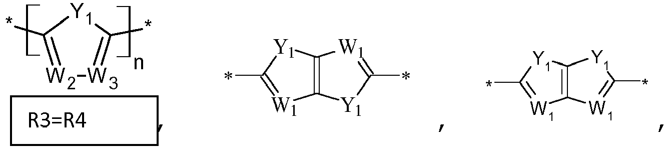 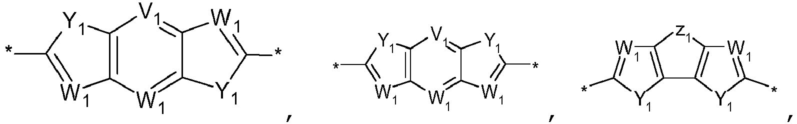 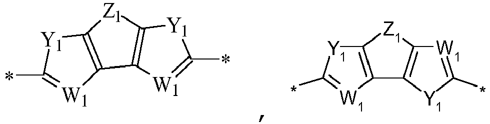
wobei Y₁ und Z₁ unabhängig ausgewählt ist aus: O, S, Se, Si(RR'), C(RR') und N(R), mit R und R' unabhängig voneinander ausgewählt aus H, C1-C26 Alkyl, linear oder verzweigt, oder Aryl mit 5-20 aromatischen Ringatomen, substituiert oder unsubstituiert, und
W₁ und V₁ unabhängig ausgewählt ist aus: N und C-R', mit R' unabhängig voneinander ausgewählt aus H, C1-C26 Alkyl, C1 -C26 O-Alkyl, C1-C26 S-Alkyl, jeweils linear oder verzweigt, oder Aryl mit 5-20 aromatischen Ringatomen, substituiert oder unsubstituiert, und
W₂ jeweils unabhängig ausgewählt ist aus N und C-R9 mit R9 unabhängig ausgewählt aus H, Halogen, C1-C26 Alkyl, C1-C26 Alkenyl, C1-C26 Alkinyl, jeweils linear oder verzweigt, OR', SR', SiR' 3, NR'2 mit R' ausgesucht aus C1-C26 Alkyl, linear oder verzweigt, oder Aryl mit 5 bis 20 aromatischen Ringatomen, substituiert oder unsubstituiert, und
W₃ jeweils unabhängig ausgewählt ist aus N und C-R10 mit R10 unabhängig ausgewählt aus H, Halogen, C1-C26 Alkyl, C1-C26 Alkenyl, C1-C26 Alkinyl, jeweils linear oder verzweigt, OR', SR', SiR'3, NR'2 mit R' ausgesucht aus C1-C26 Alkyl, linear oder verzweigt, oder Aryl mit 5 bis 20 aromatischen Ringatomen, substituiert oder unsubstituiert,
wobei R9 und R10 einen Ring bilden können, und
n = 1, 2 oder 3 ist.

19. Verbindungen nach einem der Ansprüche 12 bis 18, wobei R3 = R4 ist.

20. Verbindungen nach einem der Ansprüche 12 bis 18, wobei R1, R2, R5 und R6 H sind.

21. Verbindungen nach einem der Ansprüche 12 bis 20, wobei ihr Absorptionsmaximum bei einer Wellenlänge von 400 bis 600 nm liegt.

22. Verwendung der Verbindungen nach einem der Ansprüche 12 bis 21 in einer Solarzelle.

## Claims

1. Optoelectronic component comprising an electrode (2) and a counterelectrode (6) and at least one organic layer (4) between the electrode (2) and the counter-electrode (6), **characterized in that** the at least one organic layer (4) comprises a compound of the general formula (I) 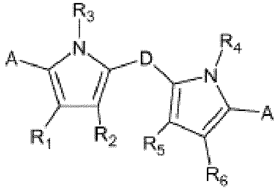
a. with R₃ and R₄ independently selected from alkyl, aryl and substituted alkyl having 1-26 carbon atoms, wherein not more than 2 C-atoms are replaced by N, O or S, or not more than 5 H-atoms are replaced by F, Cl, Br or CN,
b. R₁, R₂, R₅ and R₆ are independently selected from H, alkyl and O-alkyl, wherein R₁ and R₂ and/or R₅ and R₆ may form a ring,
c. and wherein A is selected from: 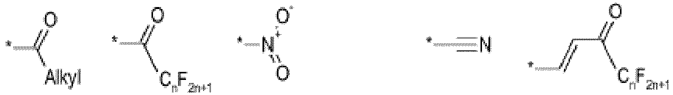 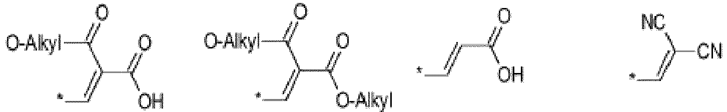 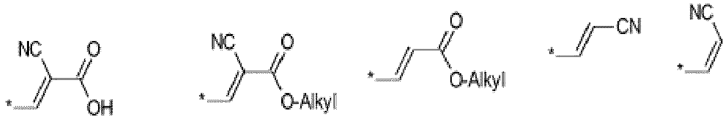 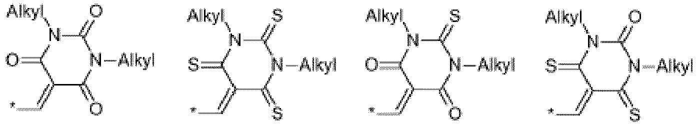 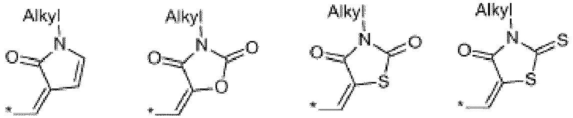 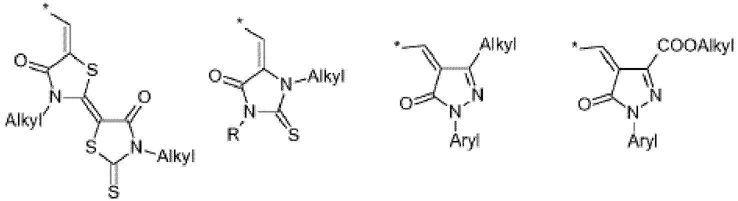 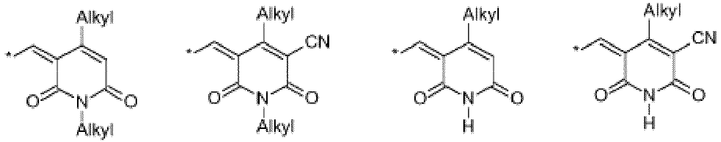 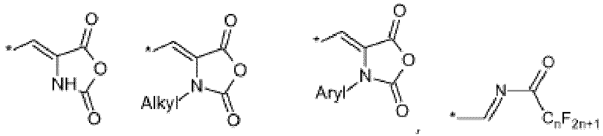 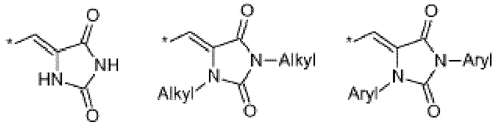
d. wherein n is an integer from 1 to 5,
e. wherein D is a unit composed of linear-conjugated aromatic 5- and/or 6-membered rings that may be fused,
f. wherein D is selected from: 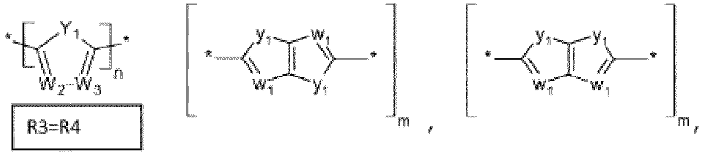 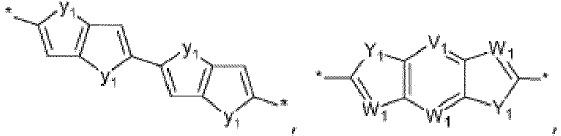 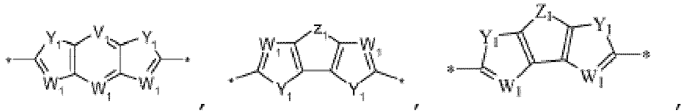 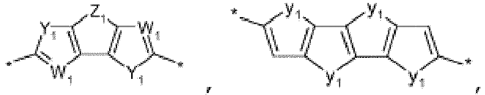 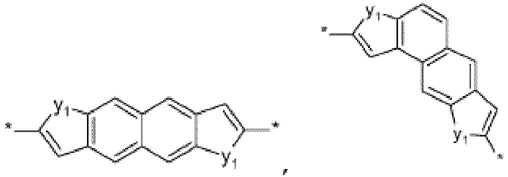
wherein Y₁ and Z₁ are independently selected from: O, S, Se, Si(RR'), C(RR') and N(R), with R and R' independently selected from H, alkyl or aryl, and
W₁ and V₁ is independently selected from: N and C-R' with R' independently selected from H, alkyl, O-alkyl, S-alkyl and aryl,
W₂ is independently selected from: N and C-R9 with R9 = H, halogen, alkyl, alkenyl, alkynyl, each linear or branched, OR', SR', SiR'₃, NR'₂, with R' selected from alkyl and aryl,
W₃ is independently selected from: N and C-R10 with R10 = H, halogen, alkyl, alkenyl, alkynyl, each linear or branched, OR', SR', SiR'₃, NR'₂, with R' selected from alkyl and aryl,
wherein R9 and R10 may form a ring, and
n = 1, 2 or 3 and m = 1 or 2.

2. Optoelectronic component according to Claim 1, wherein A is selected from: 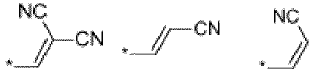 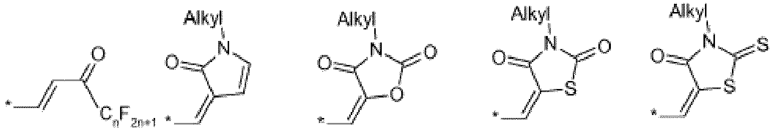 wherein n is an integer from 1 to 5.

3. Optoelectronic component according to Claim 1, wherein R₃ = R₄ = H and A is selected from

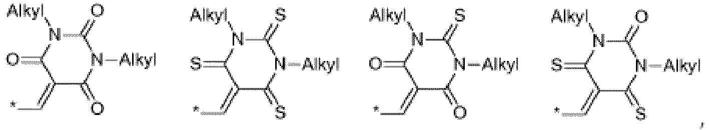

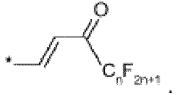

4. Optoelectronic component according to Claim 1, wherein R3 and R4 are independently alkyl, aryl or substituted alkyl having 1-26 C-atoms, wherein not more than 2 C-atoms are replaced by N, O or S or not more than 5 H-atoms are replaced by F, Cl, Br or CN, and A is selected from

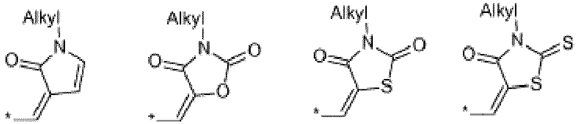

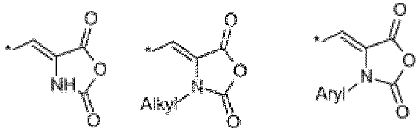

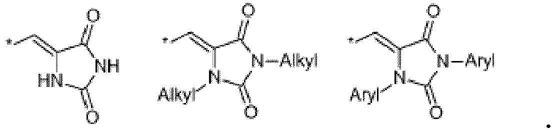

5. Optoelectronic component according to Claim 1, wherein each A is the same and is selected from cis-CH=CHCN, trans-CH=CHCN and CH=C(CN)₂.

6. Optoelectronic component according to any of Claims 1 to 5, wherein D is selected from: 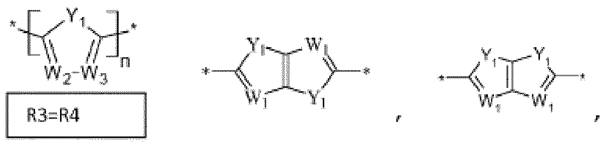 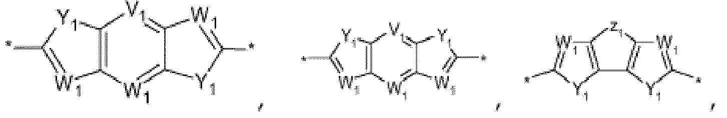 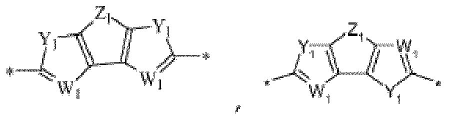
wherein Y₁ and Z₁ are independently selected from: O, S, Se, Si(RR'), C(RR') and N(R), with R and R' independently selected from H, alkyl and aryl, and
W₁ and V₁ is independently selected from: N and C-R' with R' independently selected from H, alkyl, O-alkyl, S-alkyl and aryl,
W₂ is independently selected from: N and C-R9 with R9 = H, halogen, alkyl, alkenyl, alkynyl, each linear or branched, OR', SR', SiR'₃, NR'₂, with R' selected from alkyl and aryl,
W₃ is independently selected from: N and C-R10 with R10 = H, halogen, alkyl, alkenyl, alkynyl, each linear or branched, OR', SR', SiR'₃, NR'₂, with R' selected from alkyl and aryl,
wherein R9 and R10 may form a ring, and
n = 1, 2 or 3 and m = 1 or 2.

7. Optoelectronic component according to any of Claims 1 to 6, wherein each A is the same and is selected from cis-CH=CHCN, trans-CH=CHCN and CH=C(CN)₂ and
wherein the D group is selected from: 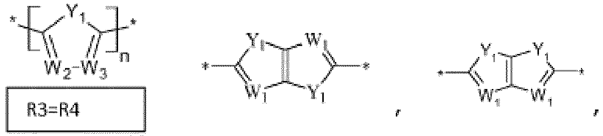 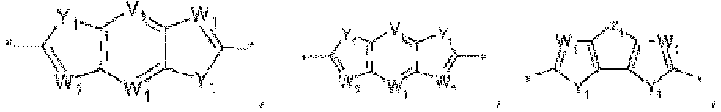 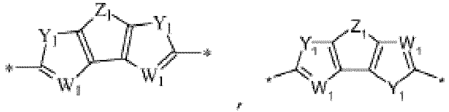
wherein Y₁ and Z₁ are independently selected from O, S, Se, Si(RR'), C(RR') and N(R), with R and R' independently selected from H, alkyl and aryl, and
W₁ and V₁ is independently selected from N and C-R' with R' independently selected from H, alkyl, O-alkyl, S-alkyl and aryl,
W₂ is independently selected from N and C-R9 with R9 independently selected from H, halogen, alkyl, alkenyl, alkynyl, each linear or branched, OR', SR', SiR'₃, NR'₂, with R' selected from alkyl and aryl,
W₃ is independently selected from N and C-R10 with R10 independently selected from H, halogen, alkyl, alkenyl, alkynyl, each linear or branched, OR', SR', SiR'₃, NR'₂, with R' selected from alkyl and aryl,
wherein R9 and R10 may form a ring, and
n = 1, 2 or 3.

8. Optoelectronic component according to any of Claims 1 to 7, wherein in the at least one compound of the general formula (I) D is selected from 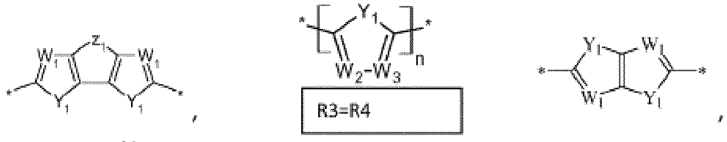 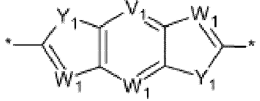
wherein Y₁ is independently selected from O, S, Se and N(R) with R selected from alkyl and aryl,
Z₁ is selected from S, Se, Si(RR'), C(RR') and N(R), with R and R' independently selected from alkyl and aryl,
W₁ and V₁ is C-R with R independently selected from H, alkyl and aryl,
W₂ is independently selected from N and C-R9 with R9 independently selected from H, halogen, alkyl, alkenyl, alkynyl, each linear or branched, OR', SR', SiR'₃, NR'₂, with R' selected from alkyl and aryl,
W₃ is independently selected from N and C-R10 with R10 independently selected from H, halogen, alkyl, alkenyl, alkynyl, each linear or branched, OR', SR', SiR'₃, NR'₂, with R' selected from alkyl and aryl,
wherein R9 and R10 may form a ring, and
n = 1, 2 or 3.

9. Optoelectronic component according to any of Claims 1 to 8, **characterized in that** in the at least one compound of the general formula (I) R3 = R4.

10. Optoelectronic component according to any of Claims 1 to 9, **characterized in that** it is an organic solar cell or a hybrid solar cell.

11. Optoelectronic component according to any of Claims 1 to 5, **characterized in that** it is an organic tandem solar cell or a multiple solar cell.

12. Compounds of the general formula (I) 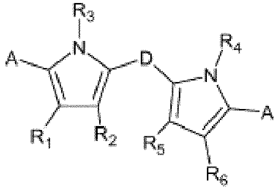
with R₃ and R₄ independently selected from alkyl, aryl and substituted alkyl having 1-26 C-atoms, wherein not more than 2 C-atoms are replaced by N, O or S, or not more than 5 H-atoms are replaced by F, Cl, Br or CN,
R1, R2, R5 and R6 are independently selected from H, alkyl and O-alkyl, wherein R1 and R2 and/or R5 and R6 may form a ring,
and wherein A is selected from: 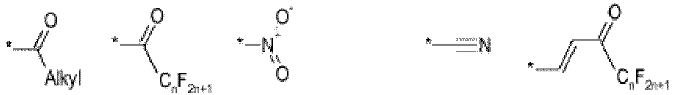 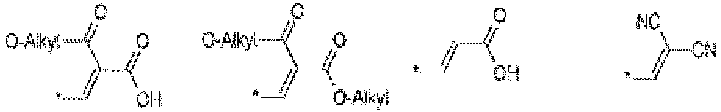 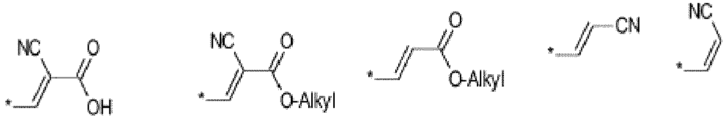 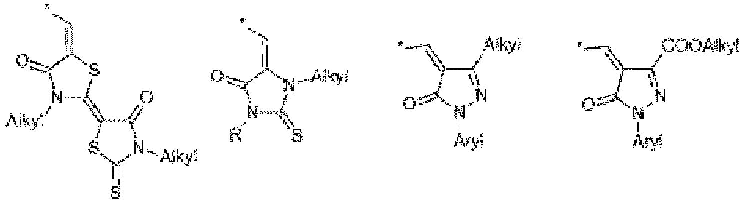 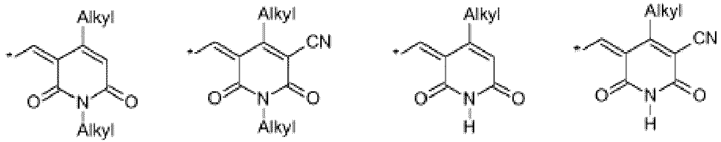 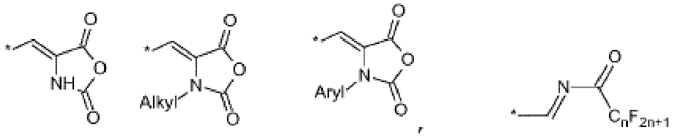 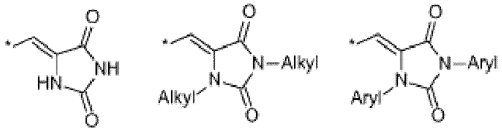
wherein n is an integer from 1 to 5,
wherein D is a unit composed of linear-conjugated aromatic 5- and/or 6-membered rings that may be fused,
wherein D is selected from: 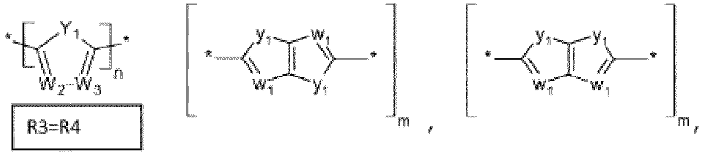 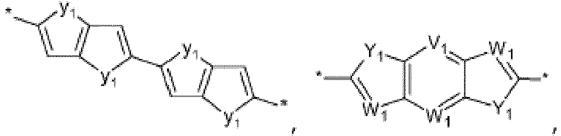 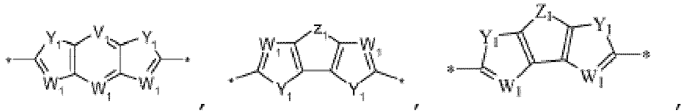 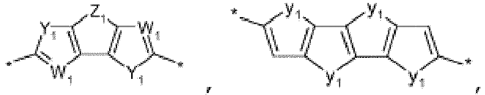 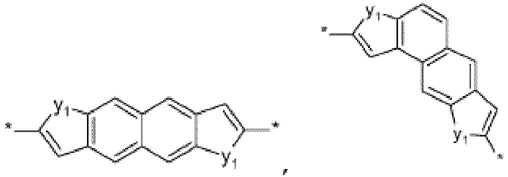
wherein Y₁ and Z₁ are independently selected from: O, S, Se, Si(RR'), C(RR') and N(R), with R and R' independently selected from H, alkyl and aryl, and
W₁ and V₁ is independently selected from: N and C-R' with R' independently selected from H, alkyl, O-alkyl, S-alkyl and aryl,
W₂ is independently selected from: N and C-R9 with R9 = H, halogen, alkyl, alkenyl, alkynyl, each linear or branched, OR', SR', SiR'₃, NR'₂, with R' selected from alkyl and aryl,
W₃ is independently selected from: N and C-R10 with R10 = H, halogen, alkyl, alkenyl, alkynyl, each linear or branched, OR', SR', SiR'₃, NR'₂, with R' selected from alkyl and aryl,
wherein R9 and R10 may form a ring, and
n = 1, 2 or 3 and m = 1 or 2.

13. Compounds according to Claim 12, wherein A is selected from: 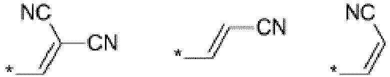 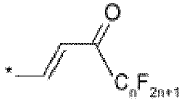 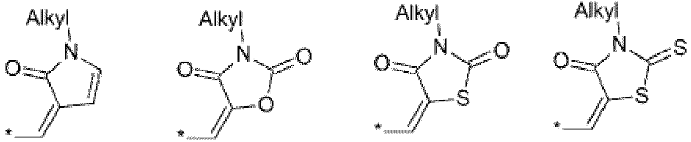 wherein n is an integer from 1 to 5.

14. Compounds according to Claim 12, wherein R3 = R4 = H and A is selected from

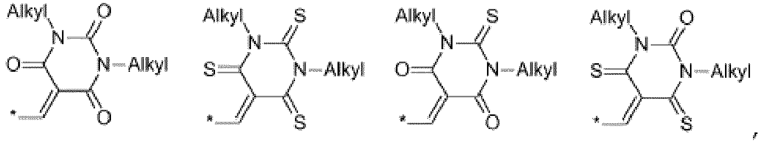

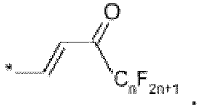

15. Compounds according to Claim 12, wherein R3 and R4 are independently alkyl, aryl or substituted alkyl having 1-26 C-atoms, wherein not more than 2 C-atoms are replaced by N, O or S or not more than 5 H-atoms are replaced by F, Cl, Br or CN, and A is selected from

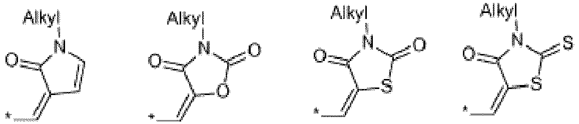

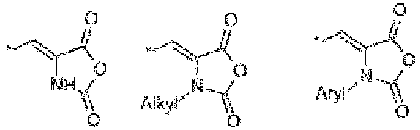

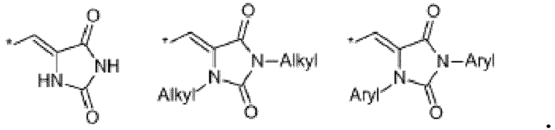

16. Compounds according to Claim 12, wherein each A is the same and is selected from cis-CH=CHCN, trans-CH=CHCN and CH=C(CN)₂.

17. Compounds according to Claim 12 or 16, wherein D is selected from: 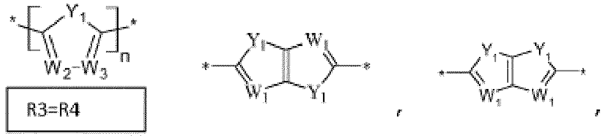 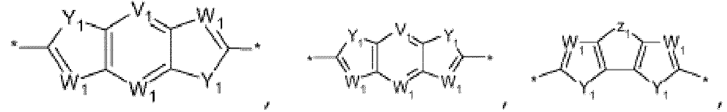 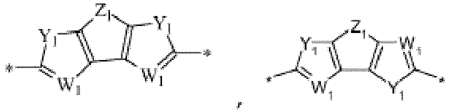
wherein Y₁ and Z₁ are independently selected from: O, S, Se, Si(RR'), C(RR') and N(R), with R and R' independently selected from H, alkyl and aryl, and W₁ and V₁ is independently selected from: N and C-R' with R' independently selected from H, alkyl, O-alkyl, S-alkyl and aryl,
W₂ is independently selected from: N and C-R9 with R9 = H, halogen, alkyl, alkenyl, alkynyl, each linear or branched, OR', SR', SiR'₃, NR'₂, with R' selected from alkyl and aryl,
W₃ is independently selected from: N and C-R10 with R10 = H, halogen, alkyl, alkenyl, alkynyl, each linear or branched, OR', SR', SiR'₃, NR'₂, with R' selected from alkyl and aryl,
wherein R9 and R10 may form a ring, and
n = 1, 2 or 3 and m = 1 or 2.

18. Compounds according to Claim 12, wherein each A is the same and is selected from cis-CH=CHCN, trans-CH=CHCN and CH=C(CN)₂,
wherein the D group is selected from: 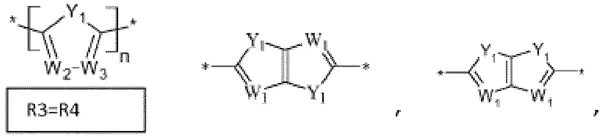 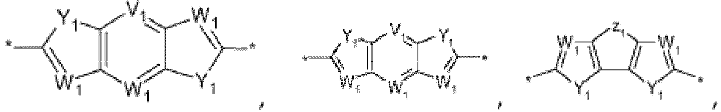 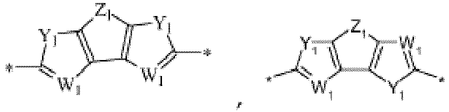
wherein Y₁ and Z₁ are independently selected from: O, S, Se, Si(RR'), C(RR') and N(R), with R and R' independently selected from H, C1-C26 alkyl, linear or branched, and aryl having 5-20 aromatic ring atoms, substituted or unsubstituted, and
W₁ and V₁ are independently selected from: N and C-R' with R' independently selected from H, C1-C26 alkyl, C1-C26 O-alkyl, C1-C26 S-alkyl, each linear or branched, and aryl having 5-20 aromatic ring atoms, substituted or unsubstituted, and
W₂ is independently selected from N and C-R9 with R9 independently selected from H, halogen, C1-C26 alkyl, C1-C26 alkenyl, C1-C26 alkynyl, each linear or branched, OR', SR', SiR'₃, NR'₂, with R' selected from C1-C26 alkyl, linear or branched, and aryl having 5 to 20 aromatic ring atoms, substituted or unsubstituted, and
W₃ is independently selected from N and C-R10 with R10 independently selected from H, halogen, C1-C26 alkyl, C1-C26 alkenyl, C1-C26 alkynyl, each linear or branched, OR', SR', SiR'₃, NR'₂, with R' selected from C1-C26 alkyl, linear or branched, and aryl having 5 to 20 aromatic ring atoms, substituted or unsubstituted,
wherein R9 and R10 may form a ring, and
n = 1, 2 or 3.

19. Compounds according to any of Claims 12 to 18, wherein R3 = R4.

20. Compounds according to any of Claims 12 to 18, wherein R1, R2, R5 and R6 are H.

21. Compounds according to any of Claims 12 to 20, having an absorption maximum at a wavelength of 400 to 600 nm.

22. Use of the compounds according to any of Claims 12 to 21 in a solar cell.

## Revendications

1. Composant optoélectronique comprenant une électrode (2) et une contre-électrode (6) et au moins une couche organique (4) entre l'électrode (2) et la contre-électrode (6), **caractérisé en ce que** l'au moins une couche organique (4) comprend un composé de formule générale (I) 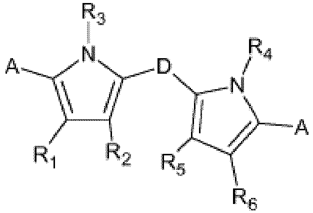
a. avec R₃ et R₄ indépendamment choisis parmi alkyle, aryle et alkyle substitué par 1 à 26 atomes de C, au maximum 2 atomes de C étant remplacés par N, O ou S ou au maximum 5 atomes de H étant remplacés par F, Cl, Br ou CN,
b. R₁, R₂, R₅ et R₆ choisis indépendamment les uns des autres parmi H, alkyle et O-alkyle, R₁ et R₂ et/ou R₅ et R₆ pouvant former un cycle,
c. et A étant choisi parmi : 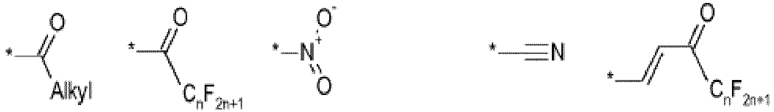 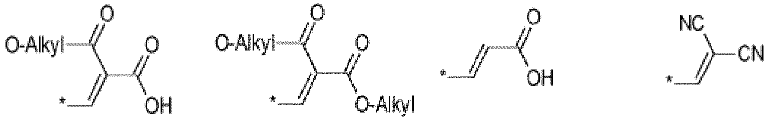 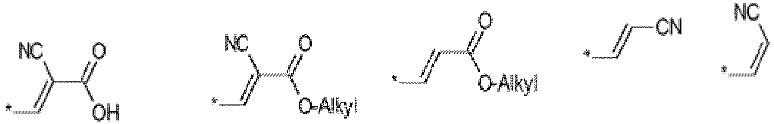 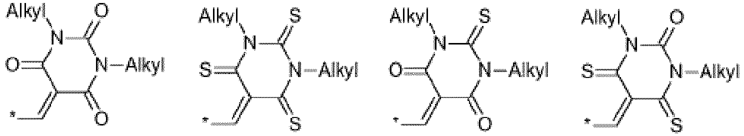 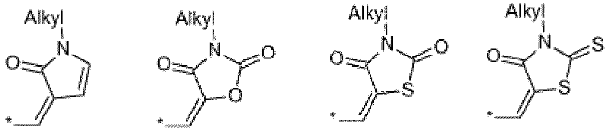 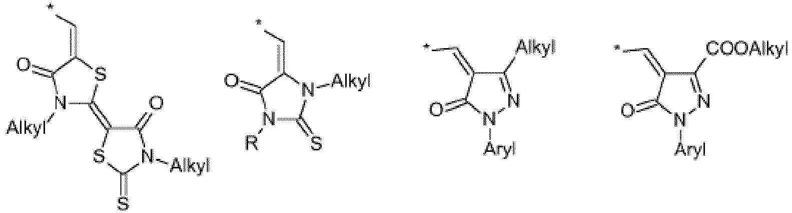 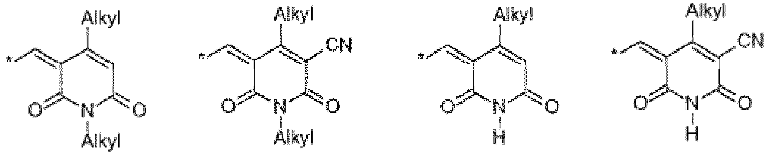 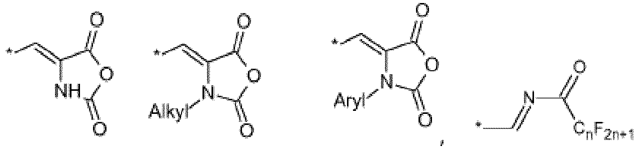 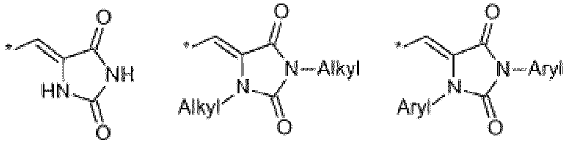
d. n étant un nombre entier de 1 à 5,
e. et D étant un motif composé de 5 et/ou 6 cycles aromatiques conjugués de manière linéaire, qui peuvent être annelés,
f. D étant choisi parmi : 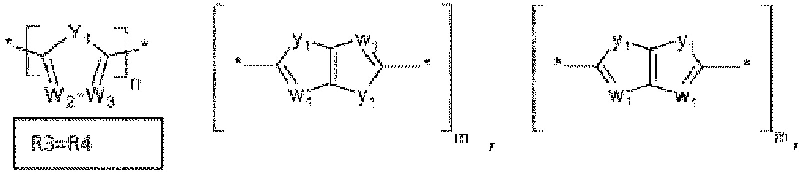 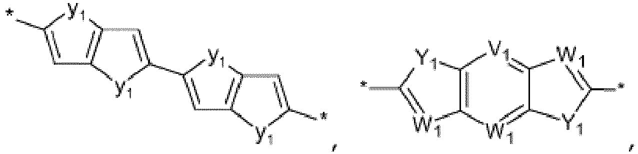 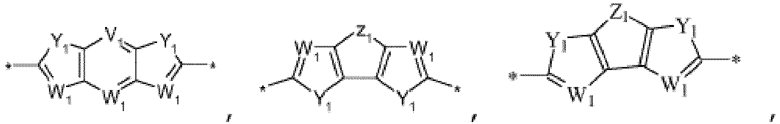 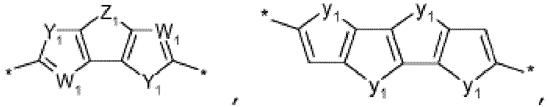 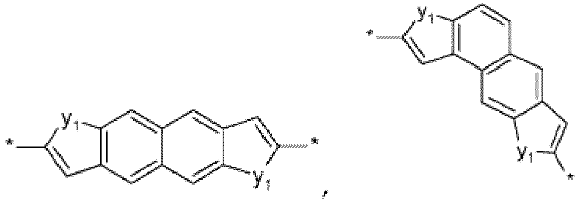
Y₁ et Z₁ étant indépendamment choisis parmi : O, S, Se, Si(RR'), C(RR') et N(R), avec R et R' choisis indépendamment l'un de l'autre parmi H, alkyle et aryle et
W₁ et V₁ étant indépendamment choisis parmi : N et C-R', avec R' choisi indépendamment l'un de l'autre parmi H, alkyle, O-alkyle, S-alkyle et aryle
W₂ étant à chaque fois indépendamment choisi parmi : N et C-R9 avec R9 = H, halogène, alkyle, alcényle, alcynyle, à chaque fois linéaire ou ramifié, OR', SR', SiR'3, NR'2 avec R' choisi parmi alkyle et aryle,
W₃ étant à chaque fois indépendamment choisi parmi : N et C-R10 avec R10 = H, halogène, alkyle, alcényle, alcynyle, à chaque fois linéaire ou ramifié, OR', SR', SiR'3, NR'2 avec R' choisi parmi alkyle et aryle,
R9 et R10 pouvant former un cycle, et
n = 1, 2 ou 3 et m = 1 ou 2.

2. Composant optoélectronique selon la revendication 1, A étant choisi parmi : 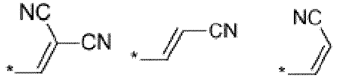 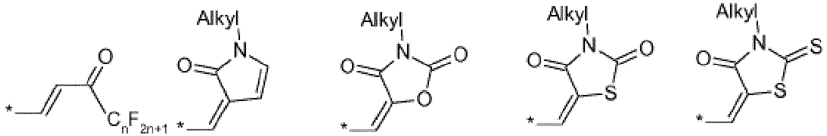 n étant un nombre entier de 1 à 5.

3. Composant optoélectronique selon la revendication 1, R₃ = R₄ = H et A étant choisi parmi

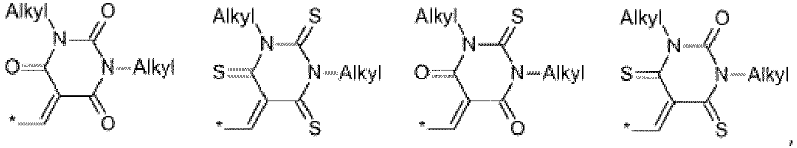

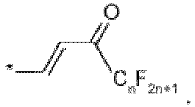

4. Composant optoélectronique selon la revendication 1, R3 et R4 étant indépendamment l'un de l'autre alkyle, aryle ou alkyle substitué par 1 à 26 atomes de C, au maximum 2 atomes de C étant remplacés par N, O ou S ou au maximum 5 atomes de H étant remplacés par F, Cl, Br ou CN et A étant choisi parmi

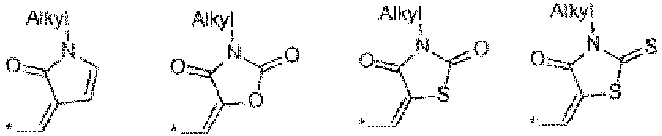

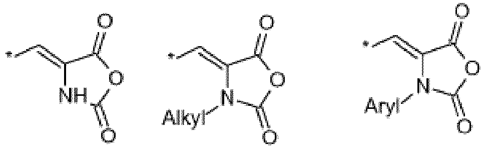

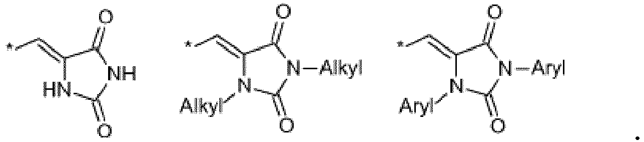

5. Composant optoélectronique selon la revendication 1, A étant à chaque fois égal et choisi parmi cis-CH=CHCN, trans-CH=CHCN et CH=C(CN)₂.

6. Composant optoélectronique selon l'une quelconque des revendications 1 à 5, D étant choisi parmi : 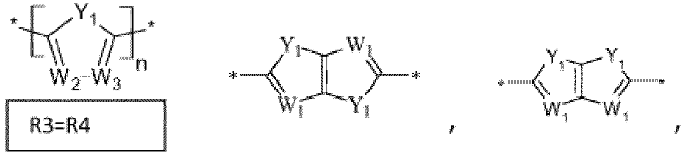 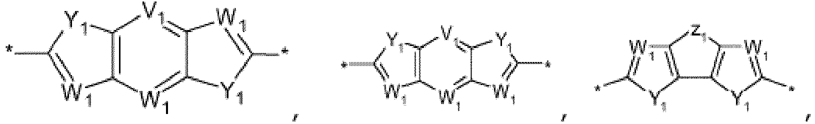 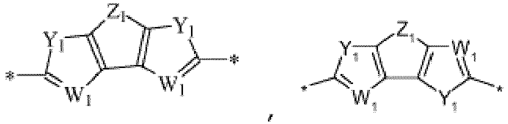
Y₁ et Z₁ étant indépendamment choisis parmi : O, S, Se, Si(RR'), C(RR') et N(R), avec R et R' choisis indépendamment l'un de l'autre parmi H, alkyle et aryle et
W₁ et V₁ étant indépendamment choisis parmi : N et C-R', avec R' choisi indépendamment l'un de l'autre parmi H, alkyle, O-alkyle, S-alkyle et aryle
W₂ étant à chaque fois indépendamment choisi parmi : N et C-R9 avec R9 = H, halogène, alkyle, alcényle, alcynyle, à chaque fois linéaire ou ramifié, OR', SR', SiR'3, NR'2 avec R' choisi parmi alkyle et aryle,
W₃ étant à chaque fois indépendamment choisi parmi : N et C-R10 avec R10 = H, halogène, alkyle, alcényle, alcynyle, à chaque fois linéaire ou ramifié, OR', SR', SiR'3, NR'2 avec R' choisi parmi alkyle et aryle,
R9 et R10 pouvant former un cycle, et
n = 1, 2 ou 3 et m = 1 ou 2.

7. Composant optoélectronique selon l'une quelconque des revendications 1 à 6, A étant à chaque fois égal et choisi parmi cis-CH=CHCN, trans-CH=CHCN et CH=C(CN)₂ et
le groupe D étant choisi parmi : 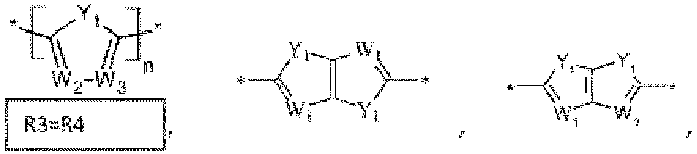 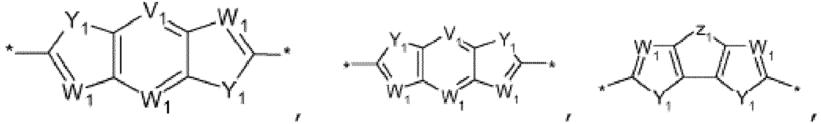 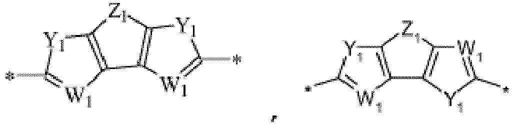
Y₁ et Z₁ étant indépendamment choisis parmi : O, S, Se, Si(RR'), C(RR') et N(R), avec R et R' choisis indépendamment l'un de l'autre parmi H, alkyle et aryle et
W₁ et V₁ étant indépendamment choisis parmi : N et C-R', avec R' choisi indépendamment l'un de l'autre parmi H, alkyle, O-alkyle, S-alkyle et aryle
W₂ étant à chaque fois indépendamment choisi parmi : N et C-R9 avec R9 indépendamment choisi parmi H, halogène, alkyle, alcényle, alcynyle, à chaque fois linéaire ou ramifié, OR', SR', SiR'₃, NR'2 avec R' choisi parmi alkyle et aryle,
W₃ étant à chaque fois indépendamment choisi parmi : N et C-R10 avec R10 indépendamment choisi parmi H, halogène, alkyle, alcényle, alcynyle, à chaque fois linéaire ou ramifié, OR', SR', SiR'₃, NR'2 avec R' choisi parmi alkyle et aryle,
R9 et R10 pouvant former un cycle, et
n = 1, 2 ou 3.

8. Composant optoélectronique selon l'une quelconque des revendications 1 à 7, dans lequel dans l'au moins un composé de formule générale (I) D est choisi parmi 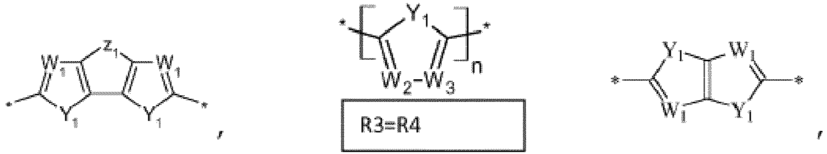 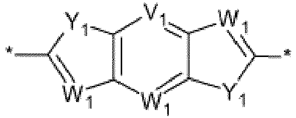
Y₁ étant indépendamment choisi parmi O, S, Se et N(R), avec R choisi parmi alkyle et aryle,
Z₁ étant choisi parmi S, Se, Si(RR'), C(RR') et N(R), avec R et R' choisis indépendamment l'un de l'autre parmi alkyle et aryle,
W₁ et V₁ étant C-R, avec R indépendamment choisi parmi H, alkyle et aryle,
W₂ étant indépendamment choisi parmi N et C-R9 avec R9 indépendamment choisi parmi H, halogène, alkyle, alcényle, alcynyle, à chaque fois linéaire ou ramifié, OR', SR', SiR'₃, NR'2 avec R' choisi parmi alkyle et aryle,
W₃ étant indépendamment choisi parmi : N et C-R10 avec R10 indépendamment choisi parmi H, halogène, alkyle, alcényle, alcynyle, à chaque fois linéaire ou ramifié, OR', SR', SiR'3, NR'2 avec R' choisi parmi alkyle et aryle,
R9 et R10 pouvant former un cycle, et
n = 1, 2 ou 3.

9. Composant optoélectronique selon l'une quelconque des revendications 1 à 8, **caractérisé en ce que** dans l'au moins un composé de formule générale (I), R3 = R4.

10. Composant optoélectronique selon l'une quelconque des revendications 1 à 9, **caractérisé en ce qu'**il s'agit d'une cellule solaire organique ou d'une cellule solaire hybride.

11. Composant optoélectronique selon l'une quelconque des revendications 1 à 5, **caractérisé en ce qu'**il s'agit d'une cellule solaire tandem organique ou d'une cellule solaire multiple organique.

12. Composés de formule générale (I) 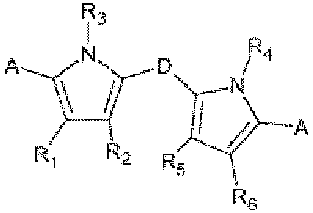
avec R₃ et R₄ indépendamment choisis parmi alkyle, aryle et alkyle substitué par 1 à 26 atomes de C, au maximum 2 atomes de C étant remplacés par N, O ou S ou au maximum 5 atomes de H étant remplacés par F, Cl, Br ou CN,
R1, R2, R5 et R6 choisis indépendamment les uns des autres parmi H, alkyle et O-alkyle, R₁ et R2 et/ou R5 et R6 pouvant former un cycle,
et A étant choisi parmi : 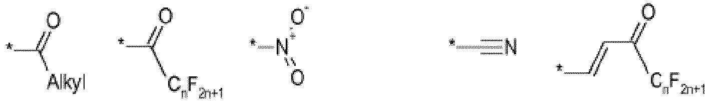 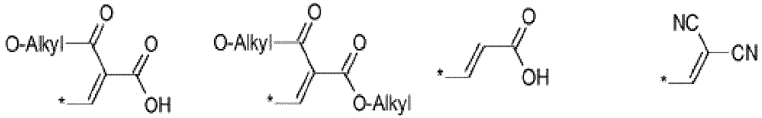 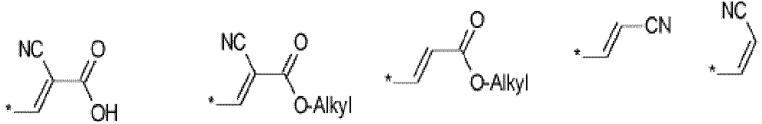 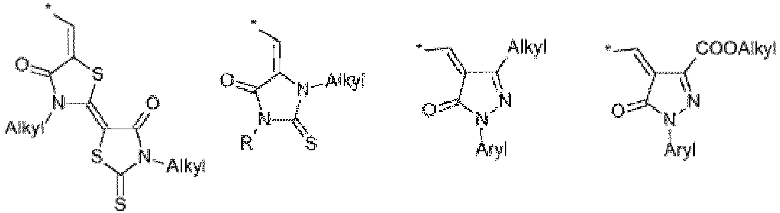 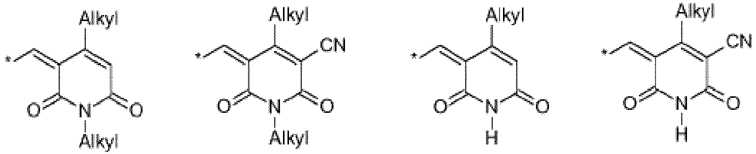 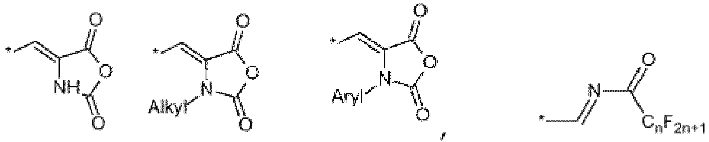 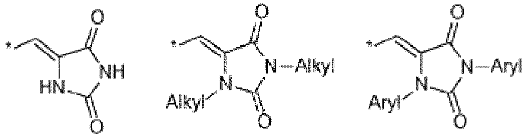
n étant un nombre entier de 1 à 5
et D étant un motif composé de 5 et/ou 6 cycles aromatiques conjugués de manière linéaire, qui peuvent être annelés,
D étant choisi parmi : 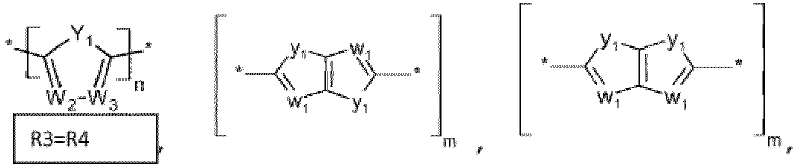 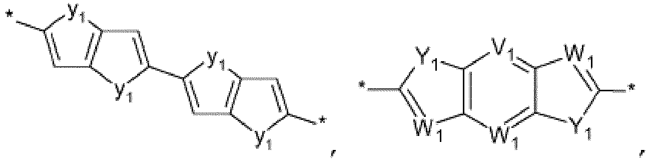 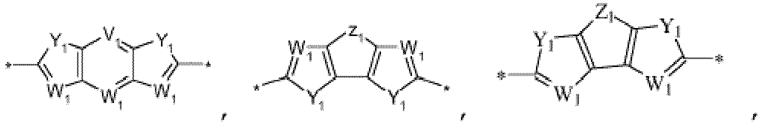 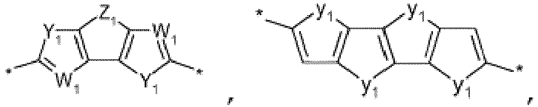 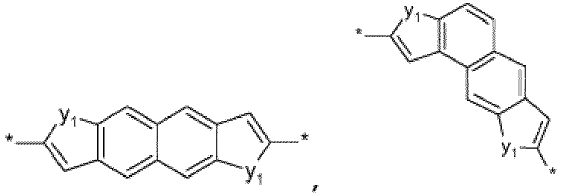
Y₁ et Z₁ étant indépendamment choisis parmi : O, S, Se, Si(RR'), C(RR') et N(R), avec R et R' choisis indépendamment l'un de l'autre parmi H, alkyle et aryle et
W₁ et V₁ étant indépendamment choisis parmi : N et C-R', avec R' choisi indépendamment l'un de l'autre parmi H, alkyle, O-alkyle, S-alkyle et aryle
W₂ étant à chaque fois indépendamment choisi parmi : N et C-R9 avec R9 = H, halogène, alkyle, alcényle, alcynyle, à chaque fois linéaire ou ramifié, OR', SR', SiR'3, NR'2 avec R' choisi parmi alkyle et aryle,
W₃ étant à chaque fois indépendamment choisi parmi : N et C-R10 avec R10 = H, halogène, alkyle, alcényle, alcynyle, à chaque fois linéaire ou ramifié, OR', SR', SiR'3, NR'2 avec R' choisi parmi alkyle et aryle,
R9 et R10 pouvant former un cycle, et
n = 1, 2 ou 3 et m = 1 ou 2.

13. Composés selon la revendication 12, A étant choisi parmi : 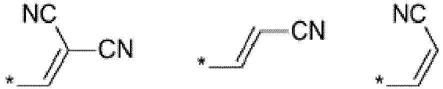 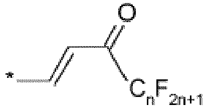 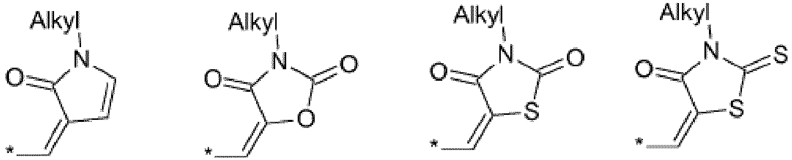 n étant un nombre entier de 1 à 5.

14. Composés selon la revendication 12, R3 = R4 = H et A étant choisi parmi

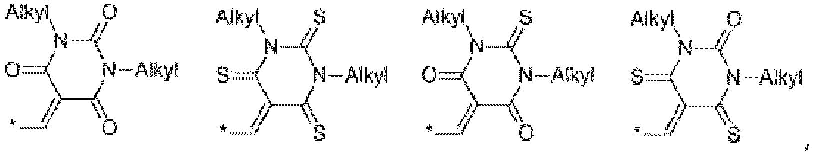

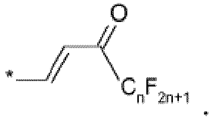

15. Composés selon la revendication 12, R3 et R4 étant indépendamment l'un de l'autre alkyle, aryle ou alkyle substitué par 1 à 26 atomes de C, au maximum 2 atomes de C étant remplacés par N, O ou S ou au maximum 5 atomes de H étant remplacés par F, Cl, Br ou CN et A étant choisi parmi

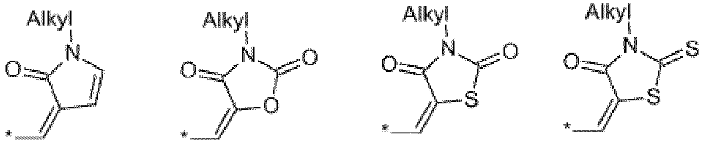

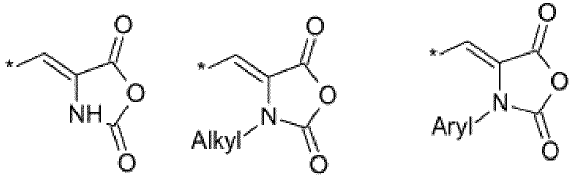

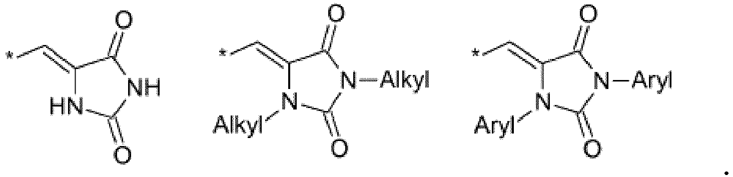

16. Composés selon la revendication 12, A étant à chaque fois égal et choisi parmi cis-CH=CHCN, trans-CH=CHCN et CH=C(CN)₂.

17. Composés selon la revendication 12 ou 16, D étant choisi parmi : 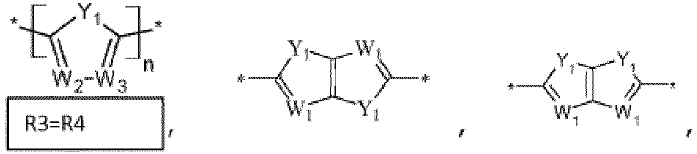 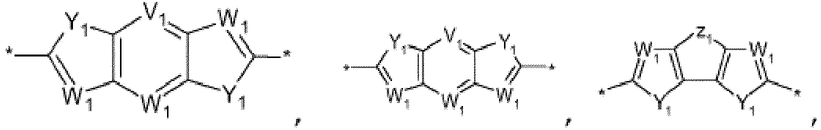 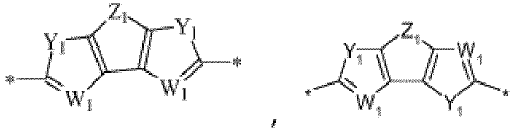
Y₁ et Z₁ étant indépendamment choisis parmi : O, S, Se, Si(RR'), C(RR') et N(R), avec R et R' choisis indépendamment l'un de l'autre parmi H, alkyle et aryle et
W₁ et V₁ étant indépendamment choisis parmi : N et C-R', avec R' choisi indépendamment l'un de l'autre parmi H, alkyle, O-alkyle, S-alkyle et aryle
W₂ étant à chaque fois indépendamment choisi parmi : N et C-R9 avec R9 = H, halogène, alkyle, alcényle, alcynyle, à chaque fois linéaire ou ramifié, OR', SR', SiR'3, NR'2 avec R' choisi parmi alkyle et aryle,
W₃ étant à chaque fois indépendamment choisi parmi : N et C-R10 avec R10 = H, halogène, alkyle, alcényle, alcynyle, à chaque fois linéaire ou ramifié, OR', SR', SiR'3, NR'2 avec R' choisi parmi alkyle et aryle,
R9 et R10 pouvant former un cycle, et
n = 1, 2 ou 3 et m = 1 ou 2.

18. Composés selon la revendication 12, A étant à chaque fois égal et choisi parmi cis-CH=CHCN, trans-CH=CHCN et CH=C(CN)₂,
le groupe D étant choisi parmi : 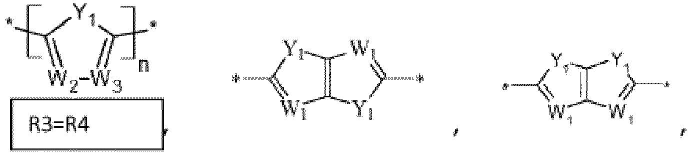 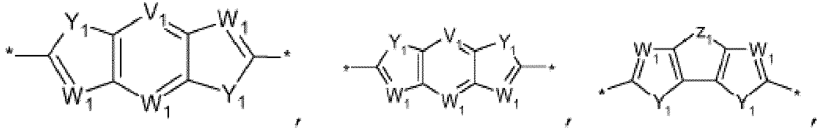 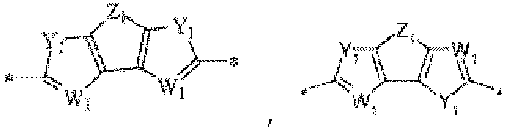
Y₁ et Z₁ étant indépendamment choisis parmi : O, S, Se, Si(RR'), C(RR') et N(R), avec R et R' choisis indépendamment l'un de l'autre parmi H, C1-C26 alkyle, linéaire ou ramifié, et aryle comportant 5 à 20 atomes de cycle aromatiques, substitué ou non substitué, et
W₁ et V₁ étant indépendamment choisis parmi : N et C-R', avec R' choisi indépendamment l'un de l'autre parmi H, C1-C26 alkyle, C1-C26 O-alkyle, C1-C26 S-alkyle, à chaque fois linéaire ou ramifié, et aryle comportant 5 à 20 atomes de cycle aromatiques, substitué ou non substitué, et W₂ étant à chaque fois indépendamment choisi parmi : N et C-R9 avec R9 indépendamment choisi parmi H, halogène, C1-C26 alkyle, C1-C26 alcényle, C1-C26 alcynyle, à chaque fois linéaire ou ramifié, OR', SR', SiR'3, NR'2 avec R' choisi parmi C1-C26 alkyle, linéaire ou ramifié, et aryle comportant 5 à 20 atomes de cycle aromatiques, substitué ou non substitué, et
W₃ étant à chaque fois indépendamment choisi parmi : N et C-R10 avec R10 indépendamment choisi parmi H, halogène, C1-C26 alkyle, C1-C26 alcényle, C1-C26 alcynyle, à chaque fois linéaire ou ramifié, OR', SR', SiR'3, NR'2 avec R' choisi parmi C1-C26 alkyle, linéaire ou ramifié, et aryle comportant 5 à 20 atomes de cycle aromatiques, substitué ou non substitué,
R9 et R10 pouvant former un cycle, et
n = 1, 2 ou 3.

19. Composés selon l'une quelconque des revendications 12 à 18, R3 = R4.

20. Composés selon l'une quelconque des revendications 12 à 18, R1, R2, R5 et R6 étant H.

21. Composés selon l'une quelconque des revendications 12 à 20, son maximum d'absorption se situant à une longueur d'onde de 400 à 600 nm.

22. Utilisation des composés selon l'une quelconque des revendications 12 à 21 dans une cellule solaire.
